(19) Europäisches Patentamt
European Patent Office
Office européen des brevets

(11) Veröffentlichungsnummer: **0 166 935**
**A2**

(12) # EUROPÄISCHE PATENTANMELDUNG

(21) Anmeldenummer: **85106145.7**

(22) Anmeldetag: **20.05.85**

(51) Int. Cl.[4]: **C 07 F 7/08**
C 07 D 249/08, C 07 C 69/712
C 07 C 79/35, C 07 C 121/75
C 07 C 103/34, C 07 C 103/365
C 07 C 103/37, C 07 C 93/06
A 01 N 31/14, A 01 N 33/06

(30) Priorität: **28.05.84 DE 3419937**

(43) Veröffentlichungstag der Anmeldung:
**08.01.86 Patentblatt 86/2**

(84) Benannte Vertragsstaaten:
**AT BE CH DE FR GB IT LI NL SE**

(71) Anmelder: **BAYER AG**
**Konzernverwaltung RP Patentabteilung**
**D-5090 Leverkusen 1 Bayerwerk(DE)**

(72) Erfinder: **Kirsten, Rolf, Dr.**
**Carl-Langhans-Strasse 27**
**D-4019 Monheim(DE)**

(72) Erfinder: **Wegler, Richard, Dr.**
**Auf dem Forst 2**
**D-5090 Leverkusen 1(DE)**

(72) Erfinder: **Eue, Ludwig, Dr.**
**Paul-Klee-Strasse 36**
**D-5090 Leverkusen 1(DE)**

(72) Erfinder: **Santel, Hans-Joachim, Dr.**
**Gerstenkamp 19**
**D-5000 Köln 80(DE)**

(72) Erfinder: **Schmidt, Robert, R., Dr.**
**Im Waldwinkel 110**
**D-5060 Bergisch-Gladbach 2(DE)**

(54) **Substituierte Diphenylether und Diphenylthioether.**

(57) Neue substituierte Diphenylether und Diphenylthioether der Formel

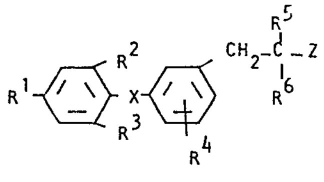

(I)

in welcher
$R^1$, $R^2$, $R^3$, $R^4$, $R^5$, $R^6$, X und Z die in der Beschreibung angegebenen Bedeutungen haben,
mehrere Verfahren zur Herstellung der neuen Stoffe und deren Verwendung als Herbizide.

EP 0 166 935 A2

BAYER AKTIENGESELLSCHAFT 5090 Leverkusen, Bayerwerk
Konzernverwaltung RP
Patentabteilung Dü/Lö-c
Ib

## Substituierte Diphenylether und Diphenylthioether

Die vorliegende Erfindung betrifft neue substituierte Diphenylether und Diphenylthioether, mehrere Verfahren zu ihrer Herstellung und ihre Verwendung als Herbizide.

Es ist bereits bekannt, daß bestimmte Phenylpropionsäure-Derivate herbizide Eigenschaften besitzen (vergl. GB-PS 1 077 194). So kann z.B. 2-Chlor-3-(4-chlorphenyl)-propionsäuremethylester zur Unkrautbekämpfung eingesetzt werden. Die Wirkung dieser Verbindung ist gut, jedoch werden bei geringen Aufwandmengen einige Unkräuter nicht immer voll erfaßt.

Es wurden nun neue substituierte Diphenylether und Diphenylthioether der Formel

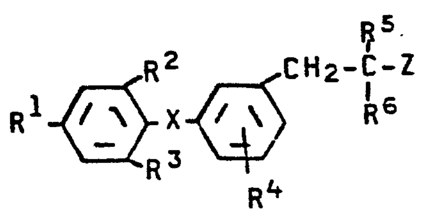

(I)

in welcher

X für Sauerstoff oder Schwefel steht,

$R^1$ für Halogen, Trihalogenmethyl, Trihalogenmethylsulfonyl, Cyano oder Nitro steht,

$R^2$ und $R^3$ unabhängig voneinander für Wasserstoff, Halogen, Trihalogenmethyl, Trihalogenmethylsulfonyl, Nitro oder Cyano stehen,

$R^4$ für Wasserstoff, Nitro oder Cyano steht,

$R^5$ und $R^6$ unabhängig voneinander für Wasserstoff, Chlor oder Brom stehen und

Z für Formyl oder den Rest der Formel

$$-\overset{X^1}{\overset{\|}{C}}-X^2-(\underset{R^8}{\overset{R^7}{C}})_m-(CH_2)_n-Y$$ steht,

in welcher

$X^1$ und $X^2$ unabhängig voneinander für Sauerstoff oder Schwefel stehen,

$R^7$ und $R^8$ unabhängig voneinander für Wasserstoff oder Alkyl mit 1 bis 4 Kohlenstoffatomen stehen,

0166935

m für 0, 1 oder 2 steht,

n für 0 oder 1 steht und

Y für Cyano, Alkoxycarbonyl, gegebenenfalls substituiertes über Stickstoff gebundenes Azolyl, gegebenenfalls substituiertes Cycloalkyl oder Cycloalkenyl sowie für die Reste der Formeln

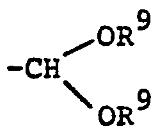 ; $-Si(CH_3)_2R^{10}$ ; 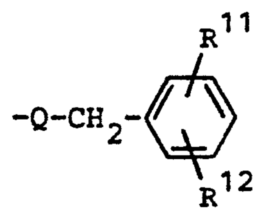 oder

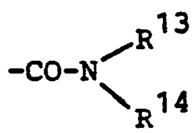 steht,

in welchen

$R^9$ für Alkyl mit 1 bis 4 Kohlenstoffatomen steht oder die beiden Reste $R^9$ gemeinsam für eine Alkylenkette mit 2 oder 3 Kohlenstoffatomen stehen,

$R^{10}$ für Alkyl mit 1 bis 4 Kohlenstoffatomen oder für Trimethylsilyloxy steht,

Q für Sauerstoff, Schwefel, SO oder $SO_2$ steht,

0166935

$R^{11}$ und $R^{12}$ unabhängig voneinander für Wasserstoff, Halogen, Alkyl mit 1 bis 4 Kohlenstoffatomen, Alkoxy mit 1 bis 4 Kohlenstoffatomen, Alkylthio mit 1 bis 4 Kohlenstoffatomen, Nitro, Cyano oder Alkoxycarbonyl mit 1 bis 4 Kohlenstoffatomen in der Alkoxygruppe stehen und

$R^{13}$ und $R^{14}$ unabhängig voneinander für Wasserstoff, für Alkyl mit 1 bis 6 Kohlenstoffatomen, für Alkenyl und Alkinyl mit jeweils 2 bis 6 Kohlenstoffatomen, für gegebenenfalls ein- bis dreifach, gleichartig oder verschieden durch Methyl oder Ethyl substituiertes Cycloalkyl oder Cycloalkenyl mit 5 bis 7 Kohlenstoffatomen, für Alkoxy mit 1 bis 6 Kohlenstoffatomen, Alkoxyalkylenoxy mit 1 bis 6 Kohlenstoffatomen in der Alkoxygruppe und 1 bis 6 Kohlenstoffatomen im Alkylenoxy-Teil, Alkoxyalkyl mit 1 bis 6 Kohlenstoffatomen in der Alkoxygruppe und 1 bis 6 Kohlenstoffatomen im Alkyl-Teil, für Halogenalkyl mit 1 bis 6 Kohlenstoffatomen und 1 bis 5 Halogenatomen, für Benzyl sowie für gegebenenfalls einfach bis dreifach, gleichartig oder verschieden durch Methyl, Ethyl, Methoxy, Methylthio, Trifluormethylthio, Fluor, Chlor und/oder Nitro substituiertes Phenyl stehen oder

$R^{13}$ und $R^{14}$ zusammen mit dem Stickstoffatom, an das sie gebunden sind, für gegebenenfalls substituiertes Piperidinyl, gegebenenfalls substituiertes Pyrrolidinyl, gegebenenfalls substituiertes Hexahydroazepinyl, gegebenenfalls substituiertes Morpholinyl, gegebenenfalls substituiertes Tetrahydrochinolyl oder gegebenenfalls substituiertes Perhydrochinolyl stehen,

gefunden.

Weiterhin wurde gefunden, daß man

a) diejenigen substituierten Diphenylether und Diphenylthioether der Formel (I),
in denen
Z für den Rest der Formel

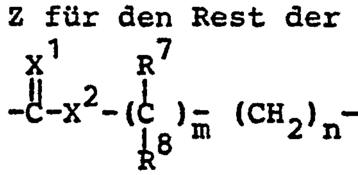 steht,

worin
$X^1$, $X^2$, Y, $R^7$, $R^8$, m und n die oben angegebenen Bedeutungen haben,

erhält, wenn man

α) (Thio)Carbonsäure-Derivate der Formel

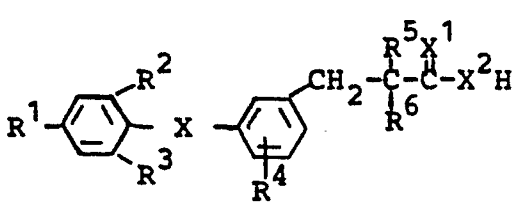 (II)

in welcher

$R^1$, $R^2$, $R^3$, $R^4$, $R^5$, $R^6$, X, $X^1$ und $X^2$ die oben angegebenen Bedeutungen haben,

mit Verbindungen der Formel

$$W-(\overset{R^7}{\underset{R^8}{\overset{|}{\underset{|}{C}}}})_m-(CH_2)_n - Y \qquad (III)$$

in welcher

Y, $R^7$, $R^8$, m und n die oben angegebenen Bedeutungen haben und

W für Chlor, Brom, Mesylat oder Tosylat steht,

gegebenenfalls in Gegenwart eines Säurebindemittels sowie gegebenenfalls in Gegenwart eines Verdünnungsmittels umsetzt,

oder

β) (Thio)Carbonsäurechloride der Formel

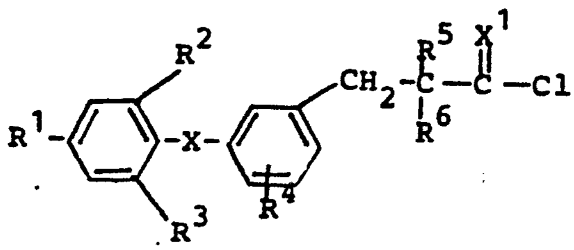

(IV)

in welcher

$R^1$, $R^2$, $R^3$, $R^4$, $R^5$, $R^6$, X und $X^1$ die oben angegebenen Bedeutungen haben,

mit Verbindungen der Formel

$$HX^2-(\overset{R^7}{\underset{R^8}{C}})_m-(CH_2)_n-Y \qquad (V)$$

in welcher

$X^2$, Y, $R^7$, $R^8$, m und n die oben angegebenen Bedeutungen haben,

gegebenenfalls in Gegenwart eines Säurebindemittels sowie gegebenenfalls in Gegenwart eines Verdünnungsmittels umsetzt,

b) die Verbindungen der Formel

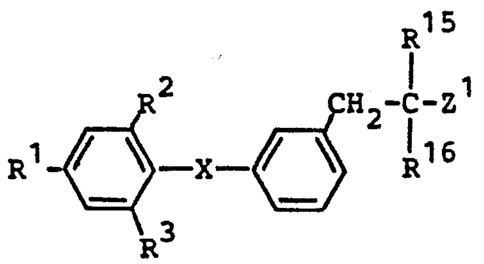

(Ia)

in welcher

$R^1$, $R^2$, $R^3$ und X die oben angegebenen Bedeutungen haben,

$R^{15}$ für Wasserstoff, Chlor oder Brom steht,

$R^{16}$ für Chlor oder Brom steht und

$Z^1$ für Formyl oder den Rest der Formel

$$-\overset{O}{\overset{\|}{C}}-O-CH_2-CH_2-Y^1$$ steht,

worin

$Y^1$ für Alkoxycarbonyl steht,

erhält, wenn man Aniline der Formel

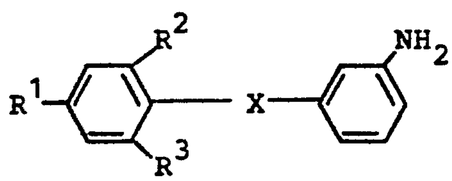

(VI)

in welcher

$R^1$, $R^2$, $R^3$ und X die oben angegebenen Bedeutungen haben,

oder Säureadditionssalze von Anilinen der Formel (VI) mit Diazotierungsmitteln in Gegenwart von Halogenwasserstoffsäuren der Formel

$HW^1$ (VII)

in welcher

$W^1$ für Chlor oder Brom steht,

und in Gegenwart eines Verdünnungsmittels umsetzt und die dabei entstehenden Diazonium-Salze der Formel

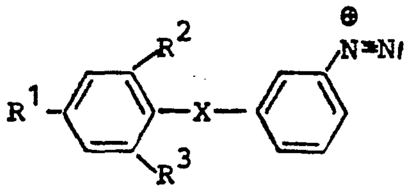 $W^{1\ominus}$ (VIII)

in welcher

$R^1$, $R^2$, $R^3$, X und $W^1$ die oben angegebenen Bedeutungen haben,

mit Verbindungen der Formel

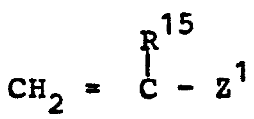 (IX)

in welcher

$R^{15}$ und $Z^1$ die oben angegebenen Bedeutungen haben,

in Gegenwart von Halogenwasserstoffsäuren der Formel

$H\ R^{16}$ (X)

in welcher

$R^{16}$ die oben angegebene Bedeutung hat,

in Gegenwart eines Katalysators und gegebenenfalls in Gegenwart eines Verdünnungsmittels umsetzt,

c) die Verbindungen der Formel

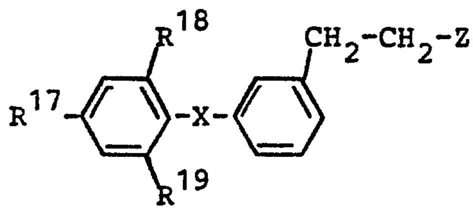

(Ib)

in welcher

X und Z die oben angegebenen Bedeutungen haben,
$R^{17}$ für Halogen oder Trihalogenmethyl steht und
$R^{18}$ und $R^{19}$ unabhängig voneinander für Wasserstoff, Halogen oder Trihalogenmethyl stehen,

erhält, wenn man Verbindungen der Formel

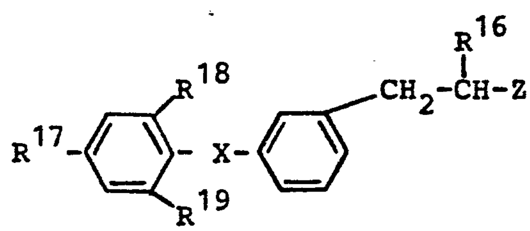

(Ic)

in welcher

$R^{16}$, $R^{17}$, $R^{18}$, $R^{19}$, X und Z die oben angegebenen Bedeutungen haben,

in Gegenwart eines Hydrierungskatalysators mit Wasserstoff gegebenenfalls in Gegenwart eines Verdünnungsmittels umsetzt,

und

d) diejenigen substituierten Diphenylether und Diphenylthioether der Formel (I),

in denen

$R^4$ für Nitro steht,

erhält, wenn man Verbindungen der Formel

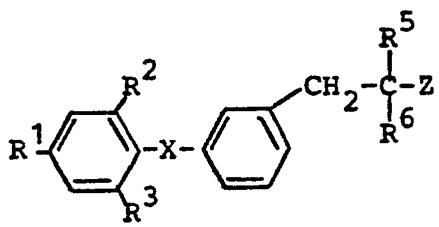

(Id)

in welcher

$R^1$, $R^2$, $R^3$, $R^5$, $R^6$, X und Z die oben angegebenen Bedeutungen haben,

mit einem Nitrierungsmittel gegebenenfalls in Gegenwart eines Katalysators sowie gegebenenfalls in Gegenwart eines Verdünnungsmittels umsetzt.

Schließlich wurde gefunden, daß sich die neuen substituierten Diphenylether und Diphenylthioether der Formel (I)

durch eine hervorragende herbizide Wirksamkeit auszeichnen.

Überraschenderweise besitzen die erfindungsgemäßen substituierten Diphenylether und Diphenylthioether der Formel (I) wesentlich bessere herbizide Eigenschaften als der aus dem Stand der Technik bekannte 2-Chlor-3-(4-chlorphenyl)-propionsäuremethylester, welcher ein hoch wirksamer Wirkstoff gleicher Wirkungsart ist. Vor allem lassen sich mit Hilfe der erfindungsgemäßen Wirkstoffe einige Ungräser, die von dem 2-Chlor-3-(4-chlorphenyl)-propionsäuremethylester nicht voll erfaßt werden, wirksam bekämpfen.

Die erfindungsgemäßen substituierten Diphenylether und Diphenylthioether sind durch die Formel (I) allgemein definiert. In dieser Formel steht X für Sauerstoff oder Schwefel.

$R^1$ steht vorzugsweise für Chlor, Brom, Trifluormethyl, Chlordifluormethyl, Trifluormethylsulfonyl, Cyano oder Nitro,

$R^2$ und $R^3$ stehen unabhängig voneinander vorzugsweise für Wasserstoff, Chlor, Brom, Trifluormethyl, Chlordifluormethyl, Trifluormethylsulfonyl, Nitro oder Cyano,

$R^4$ steht vorzugsweise für Wasserstoff, Nitro oder Cyano,

$R^5$ und $R^6$ stehen unabhängig voneinander vorzugsweise für Wasserstoff, Chlor oder Brom und

Z steht vorzugsweise für Formyl oder den Rest der Formel

$$-\overset{X^1}{\overset{\|}{C}}-X^2-(\overset{R^7}{\underset{R^8}{\overset{|}{\underset{|}{C}}}})_m-(CH_2)_n-Y,$$

worin

$X^1$ und $X^2$ unabhängig voneinander vorzugsweise für Sauerstoff oder Schwefel stehen,

$R^7$ und $R^8$ unabhängig voneinander vorzugsweise für Wasserstoff oder Methyl stehen,

m für 0, 1 oder 2 steht,

n für 0 oder 1 steht und

Y vorzugsweise für Cyano, Alkoxycarbonyl mit 1 bis 4 Kohlenstoffatomen im Alkoxyteil oder für einen über ein Ring-Stickstoffatom gebundenen Pyrazolyl-, Imidazolyl-, 1,2,4-Triazolyl-, 1,2,3-Triazolyl- oder 1,3,4-Triazolyl-Rest steht, wobei jeder dieser Azolyl-Reste ein- oder mehrfach, gleichartig oder verschieden durch Fluor, Chlor, Brom, Jod, Alkyl mit 1 bis 4 Kohlenstoffatomen, Alkoxy mit 1 bis 4 Kohlenstoffatomen und/oder Phenyl substituiert sein kann,

und

Y weiterhin vorzugweise für gegebenenfalls ein- oder mehrfach, gleichartig oder verschieden durch Alkyl mit 1 bis 4 Kohlenstoffatomen substituiertes Cycloalkyl mit 3 bis 8 Kohlenstoffatomen im Cycloalkylteil, für gegebenenfalls ein- oder mehrfach, gleichartig oder verschieden durch Alkyl mit 1 bis 4 Kohlenstoffatomen substituiertes Cycloalkenyl mit 5 bis 8 Kohlenstoffatomen im Cycloalkenylteil oder für die Reste der Formeln

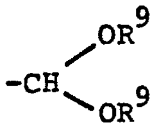 , $-Si(CH_3)_2R^{10}$ , 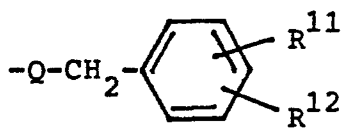

oder 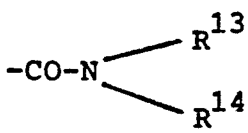 steht,

in welchen

$R^9$ vorzugsweise für Alkyl mit 1 oder 2 Kohlenstoffatomen steht oder die beiden Reste $R^9$ gemeinsam für eine Alkylenkette mit 2 oder 3 Kohlenstoffatomen stehen,

$R^{10}$ vorzugweise für Alkyl mit 1 oder 2 Kohlenstoffatomen oder für Trimethylsilyloxy steht,

Q vorzugsweise für Sauerstoff, Schwefel, SO oder $SO_2$ steht,

$R^{11}$ und $R^{12}$ unabhängig voneinander vorzugsweise für Wasserstoff, Fluor, Chlor, Brom, Jod, Alkyl mit 1 oder 2 Kohlenstoffatomen, Alkoxy mit 1 oder 2 Kohlenstoffatomen, Alkylthio mit 1 oder 2 Kohlenstoffatomen, Nitro, Cyano oder Alkoxycarbonyl mit 1 bis 2 Kohlenstoffatomen in der Alkoxygruppe stehen,

und

$R^{13}$ und $R^{14}$ unabhängig voneinander vorzugweise für Wasserstoff, Alkyl mit 1 bis 4 Kohlenstoffatomen, Alkenyl und Alkinyl mit jeweils 2 bis 4 Kohlenstoffatomen, für gegebenenfalls ein- oder zweifach, gleichartig oder verschieden durch Methyl oder Ethyl substituiertes Cycloalkyl mit 5 bis 7 Kohlenstoffatomen, für gegebenenfalls ein- oder zweifach, gleichartig oder verschieden durch Methyl oder Ethyl substituiertes Cycloalkenyl mit 5 bis 7 Kohlenstoffatomen, für Alkoxy mit 1 bis 4 Kohlenstoffatomen, Alkoxyalkylenoxy mit 1 bis 4 Kohlenstoffatomen in der Alkoxygruppe und 1 bis 4 Kohlenstoffatomen im Alkylenoxy-Teil, Alkoxyalkyl mit 1 bis 4 Kohlenstoffatomen in der Alkoxygruppe und 1 bis 4 Kohlenstoffatomen im Alkylteil, für Halogenalkyl mit 1 bis 4 Kohlenstoffatomen und 1 bis 5 Fluor-, Chlor- und/oder Brom-Atomen, für Benzyl sowie für gegebenenfalls ein- bis dreifach, gleichartig oder verschieden durch Methyl, Ethyl, Methoxy, Methylthio, Trifluormethyl, Trifluormethoxy, Trifluormethylthio, Fluor, Chlor und/oder Nitro substituiertes Phenyl stehen, oder

$R^{13}$ und $R^{14}$ zusammen mit dem Stickstoffatom, an das sie gebunden sind, vorzugsweise für Piperidinyl, Pyrrolidinyl, Hexahydroazepinyl, Morpholinyl, Tetrahydrochinolyl oder Perhydrochinolyl stehen, wobei jeder dieser heterocyclischen Reste einfach bis dreifach, gleichartig oder verschieden durch Methyl und/oder Ethyl substituiert sein kann.

Besonders bevorzugt sind diejenigen Stoffe der Formel (I), in denen

X für Sauerstoff steht,

$R^1$ für Chlor, Brom, Trifluormethyl, Chlordifluormethyl oder Nitro steht,

$R^2$ und $R^3$ unabhängig voneinander für Wasserstoff, Chlor, Brom, Trifluormethyl, Chlordifluormethyl, Nitro oder Cyano stehen,

$R^4$ für Wasserstoff oder Nitro steht,

$R^5$ und $R^6$ unabhängig voneinander für Wasserstoff, Chlor, oder Brom stehen und

Z für den Rest der Formel

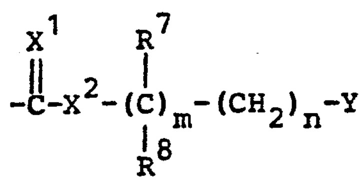

steht,

worin

$X^1$ und $X^2$ für Sauerstoff stehen,

$R^7$ und $R^8$ unabhängig voneinander für Wasserstoff oder Methyl stehen,

m für 0, 1 oder 2 steht,

n für 0 oder 1 steht und

Y für Cyano, Alkoxycarbonyl mit 1 bis 4 Kohlenstoffatomen im Alkoxyteil oder für einen über ein Ringstickstoffatom gebundenen Pyrazolyl-, Imidazolyl,-1,2,4-Triazolyl-, 1,2,3-Triazolyl- oder 1,3,4-Triazolyl-Rest steht, wobei jeder dieser Azolyl-Reste einfach oder zweifach, gleichartig oder verschieden durch Fluor, Chlor, Brom, Methyl, Ethyl, Methoxy, Ethoxy und/oder Phenyl substituiert sein kann,

und

Y weiterhin für gegebenenfalls einfach bis dreifach durch Methyl, Ethyl, n-Propyl, i-Propyl, n-Butyl, i-Butyl, sek.-Butyl und/oder tert.-Butyl substituiertes Cycloalkyl mit 5 bis 7 Kohlenstoffatomen im Cycloalkylteil, für gegebenenfalls einfach bis dreifach durch Methyl, Ethyl, n-Propyl, i-Propyl, n-Butyl, i-Butyl, sek.-Butyl und/oder tert.-Butyl substituiertes Cycloalkenyl mit 5 bis 7 Kohlenstoffatomen und 1 oder 2 Doppelbindungen im Cycloalkenyl-Teil steht oder für die Reste der Formeln

$-Si(CH_3)_2R^{10}$, 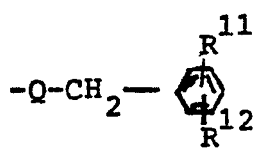 oder

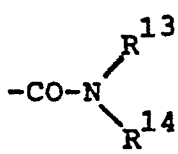

steht,

in welchen

$R^{10}$ für Methyl, Ethyl oder Trimethylsilyloxy steht,

Q für Sauerstoff oder Schwefel steht,

$R^{11}$ und $R^{12}$ unabhängig voneinander für Wasserstoff, Fluor, Chlor, Brom, Methyl, Ethyl, Methoxy, Ethoxy, Methylthio, Ethylthio, Nitro, Cyano, Methoxycarbonyl oder Ethoxycarbonyl stehen,

und

$R^{13}$ und $R^{14}$ unabhängig voneinander für Wasserstoff, Alkyl mit 1 bis 4 Kohlenstoffatomen, Alkenyl und Alkinyl mit jeweils 2 bis 4 Kohlenstoffatomen, für gegebenenfalls ein- oder zweifach, gleichartig oder verschieden durch Methyl oder Ethyl substituiertes Cycloalkyl mit 5 bis 7 Kohlenstoffatomen, für gegebenenfalls ein- oder zweifach, gleichartig oder verschieden durch Methyl oder Ethyl substituiertes Cycloalkenyl mit 5 bis 7 Kohlenstoffatomen, für Alkoxy mit 1 bis 4 Kohlenstoffatomen, Alkoxyalkylenoxy mit 1 bis 4 Kohlenstoffatomen in der Alkoxygruppe und 1 bis 4 Kohlenstoffatomen im Alkylenoxy-Teil, Alkoxyalkyl mit 1 bis 4 Kohlenstoffatomen in der Alkoxygruppe und 1 bis 4 Kohlenstoffatomen im Alkylteil, für Halogenalkyl mit 1 bis 4 Kohlenstoffatomen und 1 bis 5 Fluor-, Chlor- und/oder Brom-Atomen, für Benzyl sowie für gegebenenfalls ein- bis dreifach, gleichartig oder verschieden durch Methyl, Ethyl, Methoxy, Methylthio, Trifluormethyl, Trifluormethoxy, Trifluormethylthio, Fluor, Chlor und/oder Nitro substituiertes Phenyl stehen,

oder

$R^{13}$ und $R^{14}$ zusammen mit dem Stickstoffatom, an das sie gebunden sind, für Piperidinyl, Pyrrolidinyl, Hexahydroazepinyl, Morpholinyl, Tetrahydrochinolyl oder Perhydrochinolyl stehen, wobei jeder dieser heterocyclischen Reste einfach bis dreifach, gleichartig oder verschieden durch Methyl und/oder Ethyl substituiert sein kann.

Verwendet man 3-/3-(2-Chlor-4-trifluormethyl-phenoxy)-phenyl7-propionsäure und Chlormethyltrimethylsilan als Ausgangsstoffe, so kann der Verlauf des erfindungsgemäßen Verfahrens (a, Variante α) durch das folgende Formelschema wiedergegeben werden:

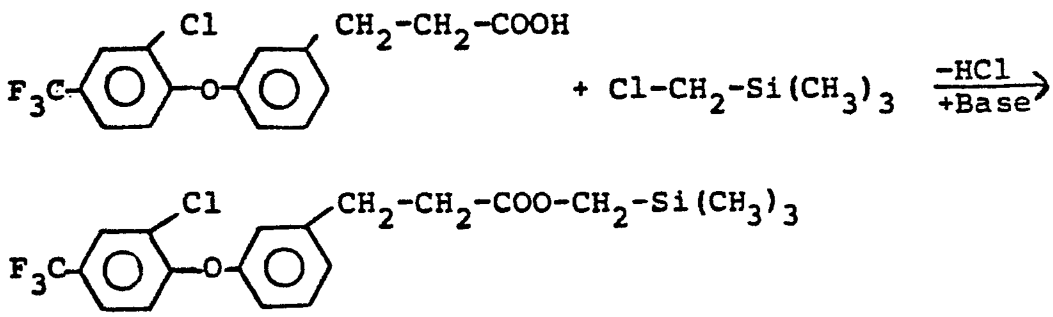


Verwendet man 3-/3-(2,6-Dichlor-4-trifluormethyl-phenoxy)-phenyl7-propionsäurechlorid und Ethylenglykol-mono-benzylether als Ausgangstoffe, so kann der Verlauf des erfindungsgemäßen Verfahrens (a, Variante β) durch das folgende Formelschema wiedergegeben werden:

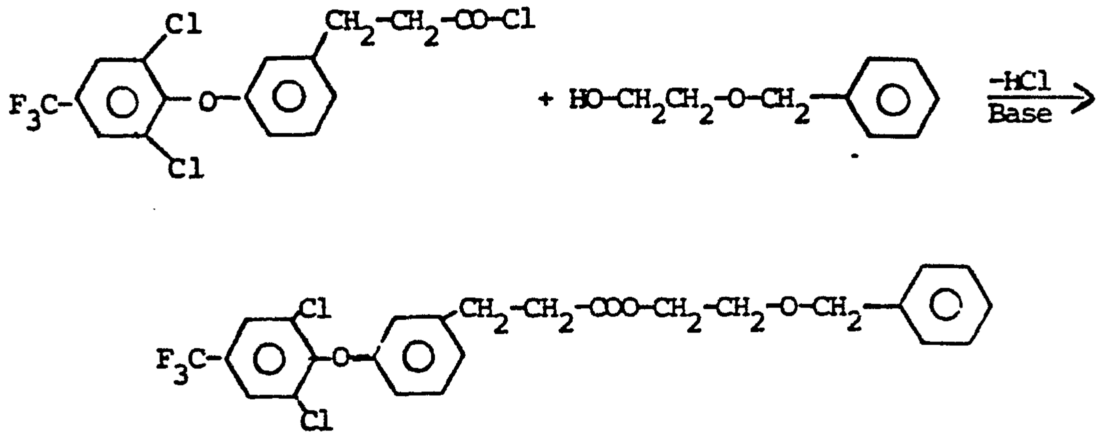


Verwendet man 3-(2-Cyano-4-trifluormethyl-phenoxy)-anilin als Ausgangsstoff, Natriumnitrit und Salzsäure als Reaktionskomponenten und setzt das dabei entstehende Diazoniumsalz mit 3-Acryloyl-propionsäuremethylester und Salzsäure in Gegenwart von Kupfer-(I)-chlorid und Kupfer-(II)-chlorid um, so kann der Verlauf des erfindungsgemäßen Verfahrens (b) durch das folgende Formelschema wiedergegeben werden:

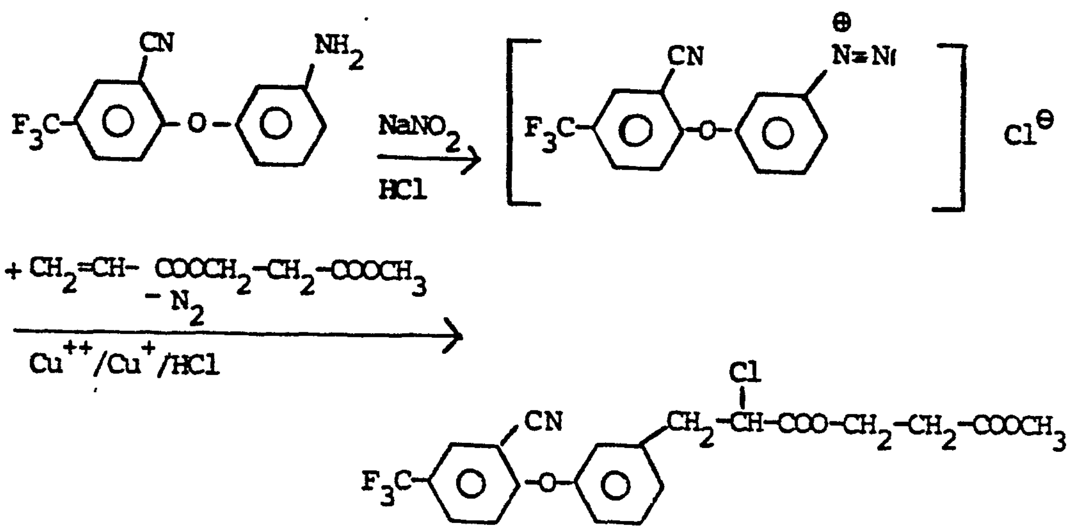

Verwendet man 2-Chlor-3-[3-(2-chlor-4-trifluormethyl-phenoxy)-phenyl]-propionsäure-2-(ethoxycarbonyl)-ethylester als Ausgangsstoff und hydriert mit Wasserstoff in Gegenwart von Raney-Nickel als Katalysator, so kann der Verlauf des erfindungsgemäßen Verfahrens (c) durch das folgende Formelschema wiedergegeben werden:

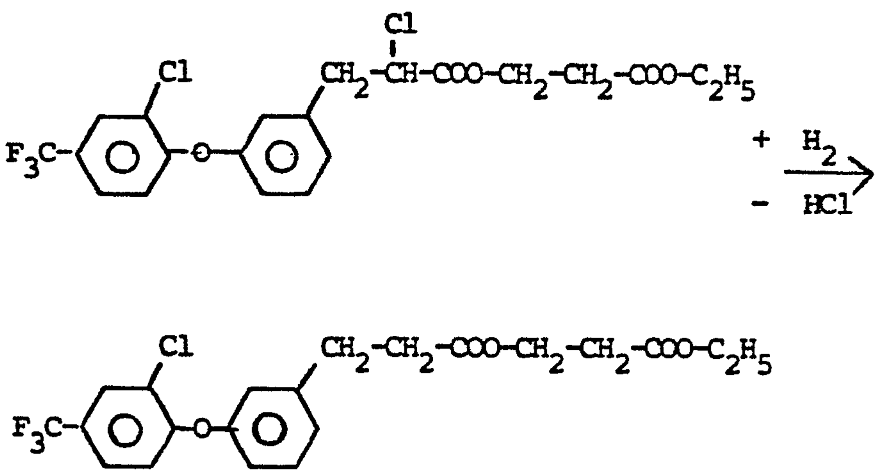


Verwendet man 2-Chlor-3-[3-(4-nitro-2-trifluormethyl-phenoxy)-phenyl]-propionsäure-2-(methoxycarbonyl)ethylester als Ausgangsstoff, Salpetersäure als Reaktionskomponente, Essigsäureanhydrid und Eisessig als Verdünnungsmittel, so kann der Verlauf des erfindungsgemäßen Verfahrens (d) durch das folgende Formelschema wiedergegeben werden:

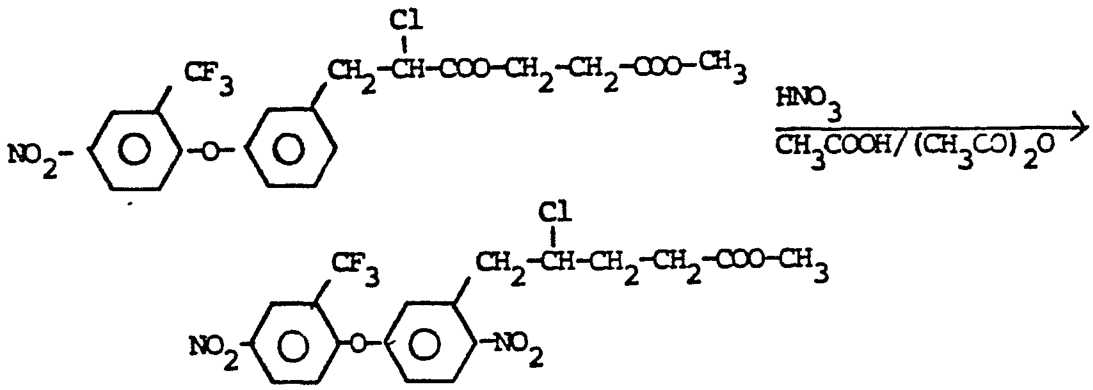


Die beim erfindungsgemäßen Verfahren (a, Variante α) als Ausgangsstoffe benötigten (Thio)Carbonsäure-Derivate sind durch die Formel (II) definiert. In dieser Formel haben $R^1$, $R^2$, $R^3$, $R^4$, $R^5$, $R^6$, X, $X^1$ und $X^2$ vorzugsweise diejenigen Bedeutungen, die bereits im Zusammenhang mit der Beschreibung der erfindungsgemäßen Stoffe der Formel (I) vorzugsweise für diese Reste genannt wurden.

Die (Thio)-Carbonsäure-Derivate der Formel (II) sind zum Teil neu. Sie lassen sich herstellen, indem man zum Beispiel Aniline der Formel

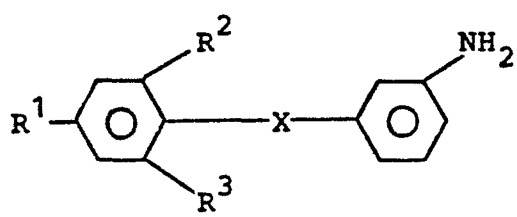

(VI)

in welcher

$R^1$, $R^2$, $R^3$ und X die oben genannten Bedeutungen haben,

oder

Säureadditionssalze von Anilinen der Fomel (VI), wie zum Beispiel die Hydrochloride oder Hydrobromide, mit Diazotierungsmitteln, wie zum Beispiel Natrium- oder Kalium-Nitrit, in Gegenwart von Halogenwasserstoffsäuren der Formel

$$HW^1 \qquad \text{(VII)}$$

in welcher

$W^1$ die oben angegebene Beudeutung hat,

und in Gegenwart eines Verdünnungsmittels, wie z.B. Aceton, bei Temperaturen zwischen -15°C und +80°C umsetzt und die dabei entstehenden Diazoniumsalze der Formel

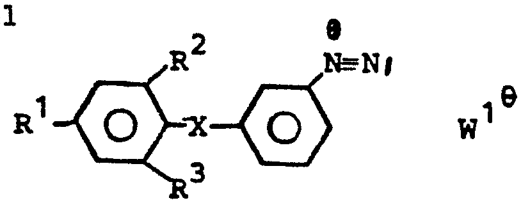

in welcher

$R^1$, $R^2$, $R^3$, X und $W^1$ die oben angegebenen Bedeutungen haben,

mit Acrylsäure-Derivaten der Formel

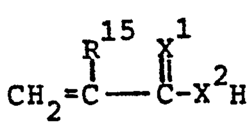

(XI)

in welcher

$R^{15}$, $X^1$ und $X^2$ die oben angegebenen Bedeutungen haben,

in Gegenwart von Halogenwasserstoffsäuren der Formel

$$HR^{16} \qquad (X)$$

in welcher

$R^{16}$ die oben angegebene Bedeutung hat,

in Gegenwart eines Katalysators, wie z.B. Kupfer-I- und Kupfer-II-chlorid, und gegebenenfalls in Gegenwart eines Verdünnungsmittels, wie z.B. Wasser und/oder Aceton, bei Temperaturen zwischen 10°C und 60°C umsetzt und gegebenenfalls die dabei entstehenden Phenylpropionsäure-Derivate der Formel

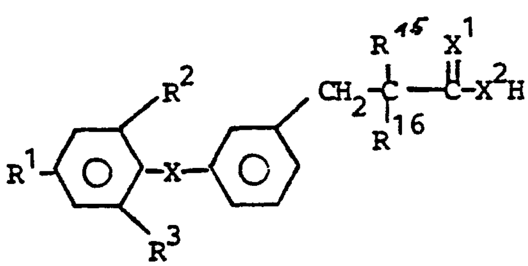

(IIa)

in welcher

$R^1$, $R^2$, $R^3$, $R^{15}$, $R^{16}$, X, $X^1$ und $X^2$ die oben angegebenen Bedeutungen haben,

in Gegenwart eines Hydrierungskatalysators, wie z.B. Raney-Nickel, in Gegenwart von Wasserstoff und gegebenenfalls in Gegenwart eines Verdünnungsmittels, wie z.B. Methanol, bei Temperaturen zwischen 20°C und 80°C und

bei einem Druck von 60 atm bis 120 atm hydriert, und gegebenenfalls die dabei entstehenden Verbindungen der Formel

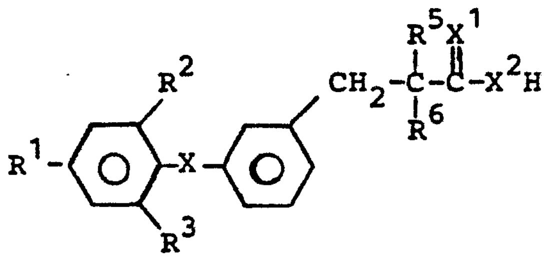

(IIb)

in welcher

$R^1$, $R^2$, $R^3$, $R^5$, $R^6$, X, $X^1$ und $X^2$ die oben angegebenen Bedeutungen haben,

mit einem Nitrierungsmittel, wie z.B. Kupfernitrat oder konzentrierter Salpetersäure, gegebenenfalls in Gegenwart eines Katalysators, wie z.B. Essigsäure, und gegebenenfalls in Gegenwart eines Verdünnungsmittels, wie z.B. Essigsäureanhydrid, bei Temperaturen zwischen -20°C und +60°C umsetzt (vergleiche Herstellungsbeispiel).

Die weiterhin für das erfindungsgemäße Verfahren (a, Variante α), zu verwendenden Verbindungen sind durch die Formel (III) definiert. In dieser Formel haben $R^7$, $R^8$, Y, m und n vorzugsweise diejenigen Bedeutungen, die bereits in Zusammenhang mit der Beschreibung der erfindungsgemäßen Stoffe der Formel (I) vorzugsweise für diese Reste bzw. Indices genannt wurden. W steht in dieser Formel für Chlor, Brom, Mesylat oder Tosylat.

Die Verbindungen der Formel (III) sind bekannt oder

0166935

lassen sich in einfacher Weise nach bekannten Methoden herstellen.

Die beim erfindungsgemäßen Verfahren (a, Variante $\beta$) als Ausgangsstoffe benötigten (Thio)Carbonsäurechloride sind durch die Formel (IV) definiert. In dieser Formel haben $R^1$, $R^2$, $R^3$, $R^4$, $R^5$, $R^6$, X und $X^1$ vorzugsweise diejenigen Bedeutungen, die bereits im Zusammenhang mit der Beschreibung der erfindungsgemäßen Stoffe der Formel (I) vorzugsweise für diese Reste genannt wurden.

Die (Thio)Carbonsäurechloride der Formel (IV) sind teilweise neu. Sie lassen sich herstellen, indem man (Thio)-Carbonsäuren der Formel (II) mit Chlorierungsmitteln, wie zum Beispiel Thionylchlorid oder Phosphortrichlorid, gegebenenfalls in Gegenwart eines Katalysators, wie z.B. Dimethylformamid oder Triphenylphosphinoxid, in Gegenwart eines inerten Verdünnungsmittels, wie zum Beispiel Methylenchlorid, Toluol oder Dichlorethan, bei Temperaturen zwischen 10°C und 140°C umsetzt (vergleiche Herstellungsbeispiel).

Die weiterhin für das erfindungsgemäße Verfahren (a, Variante $\beta$) zu verwendenden Verbindungen sind durch die Formel (V) definiert. In dieser Formel haben $X^2$, Y, $R^7$, $R^8$, m und n vorzugsweise diejenigen Bedeutungen, die bereits im Zusammenhang mit der Beschreibung der erfindungsgemäßen Stoffe der Formel (I) vorzugsweise für diese Reste bzw. Indices genannt wurden.

Die Verbindungen der Formel (V) sind bekannt oder lassen sich in einfacher Weise nach bekannten Methoden herstellen.

Als Säureakzeptoren können bei dem erfindungsgemäßen Verfahren (a) sowohl in der Variante α als auch in der Variante β alle üblicherweise für derartige Umsetzungen verwendbaren Säurebindemittel eingesetzt werden. Vorzugsweise in Frage kommen Alkalihydroxide, Erdalkalihydroxide und -oxide, wie z.B. Natrium- und Kaliumhydroxid, Calciumhydroxid und Calciumoxid, Alkalicarbonate und -alkoholate, wie Natrium- und Kaliumcarbonat, Natrium- und Kaliummethylat bzw. -ethylat, ferner aliphatische, aromatische oder herocyclische Amine, beispielsweise Triethylamin, Trimethylamin, Dimethylanilin, Dimethylbenzylamin, 1,5-Diazabicyclo/4,3,0/non-5-en (DBN), 1,8-Diazabicyclo/5,4,0/-undec-7-en (DBU) und Pyridin.

Als Verdünnungsmittel kommen bei der Durchführung des erfindungsgemäßen Verfahrens (a) sowohl in der Variante α als auch in der Variante β alle inerten organischen Solventien in Betracht. Vorzugsweise verwendbar sind aliphatische und aromatische, gegebenenfalls halogenierte Kohlenwasserstoffe, wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether, wie Diethyl- und Dibutylether, Glycoldimethylether und Diglycoldimethylether, Tetrahydrofuran und Dioxan, Ketone, wie Aceton, Methyl-ethyl-, Methyl-isopropyl- und Methylisobutyl-keton, Ester, wie z. B. Essigsäure-methylester und ethylester, Nitrile, wie z.B. Acetonitril und Propionitril, Amide, wie z.B. Dimethylformamid, Dimethylacetamid und N-Methyl-pyrrolidon, sowie Dimethylsulfoxid, Tetramethylensulfon und Hexamethylphosphorsäuretriamid.

Die Reaktionstemperaturen können bei dem erfindungsgemässen Verfahren (a) sowohl in der Variante $\alpha$ als auch in der Variante $\beta$ innerhalb eines größeren Bereiches variiert werden. Im allgemeinen arbeitet man jeweils bei Temperaturen zwischen -20°C und +160°C, vorzugsweise zwischen 0°C und +140°C.

Das erfindungsgemäße Verfahren (a) wird sowohl in der Variante $\alpha$ als auch in der Variante $\beta$ im allgemeinen unter Normaldruck durchgeführt. Es ist jedoch auch möglich, unter erhöhtem oder vermindertem Druck zu arbeiten.

Zur Durchführung des erfindungsgemäßen Verfahrens (a, Varianten $\alpha$ und $\beta$ ) werden die Ausgangsstoffe der Formeln (II) und (III) bzw. (IV) und (V), im allgemeinen in angenähert äquimolaren Mengen eingesetzt. Es ist jedoch auch möglich, eine der beiden jeweils eingesetzten Komponenten in einem größeren Überschuß zu verwenden. Die Reaktionen werden im allgemeinen in einem geeigneten Verdünnungsmittel in Gegenwart eines Säureakzeptors durchgeführt, und das Reaktionsgemisch wird mehrere Stunden bei der jeweils erforderlichen Temperatur gerührt. Die Aufarbeitung erfolgt jeweils nach üblichen Verfahren.

Die beim erfindungsgemäßen Verfahren (b) als Ausgangsstoffe benötigten Aniline sind durch die Formel (VI)

definiert. In dieser Formel stehen $R^1$, $R^2$, $R^3$ und X vorzugsweise für diejenigen Bedeutungen, die bereits im Zusammenhang mit der Beschreibung der erfindungsgemäßen Stoffe der Formel (I) vorzugsweise für diese Reste genannt wurden. Als Säureadditions-Salze der Aniline der Formel (VI) kommen die Hydrohalogenide, wie z.B. Hydrochloride und -bromide, in Frage. Die Aniline der Formel (VI) und deren Säureadditionssalze sind bekannt oder lassen sich nach bekannten Methoden herstellen (vgl. DE-OS 2 652 810, EP-OS 0 029 128 und EP-OS 0 027 965).

Die bei dem erfindungsgemäßen Verfahren (b) als Reaktionskomponenten benötigten Verbindungen sind durch die Formel (IX) definiert. In dieser Formel steht $R^{15}$ für Wasserstoff, Chlor oder Brom. $Z^1$ steht für Formyl oder für den Rest der Formel

$-\overset{O}{\overset{\|}{C}}-O-CH_2-CH_2-Y^1$, worin $Y^1$ vorzugsweise für Alkoxycarbonyl mit 1 bis 4 Kohlenstoffatomen im Alkoxyteil steht.

Die Verbindungen der Formel (IX) sind bekannt oder lassen sich nach im Prinzip bekannten Methoden herstellen (vgl. EP-OS 0 00 3045 und Derwent Ref. Nr. 40615 S).

Als Katalysatoren können bei dem erfindungsgemäßen Verfahren (b) alle üblicherweise für derartige Umsetzungen mit Diazoniumsalzen verwendbaren Katalysatoren eingesetzt werden. Vorzugsweise in Frage kommen Kupfer-Pulver, Kupfer-I-chlorid und/oder Kupfer-II-chlorid. Als Verdünnungsmittel kommen bei der Durchführung des erfindungsgemäßen Verfahrens (b) alle inerten, hydrophilen organischen Solventien und Wasser in Betracht. Vorzugsweise verwendbar sind Ether, wie Glycoldimethylether und Diglycoldimethylether, Tetrahydrofuran und Dioxan, außerdem Ketone, wie Aceton, Methyl-ethyl-, Methyl-isopropyl- und Methyl-isobutyl-keton. Das erfindungsgemäße Verfahren (b) wird bevorzugt unter Verwendung von Gemischen aus organischen Solventien und Wasser durchgeführt.

Die Reaktionstemperaturen können bei der Umsetzung nach dem erfindungsgemäßen Verfahren (b) innerhalb eines größeren Bereiches variiert werden. Im allgemeinen arbeitet man bei Temperaturen zwischen -15°C und +80°C, vorzugsweise zwischen -5°C und +60°C.

Das erfindungsgemäße Verfahren (b) wird im allgemeinen unter Normaldruck durchgeführt. Es ist jedoch auch möglich, unter erhöhtem oder vermindertem Druck zu arbeiten.

Zur Durchführung des erfindungsgemäßen Verfahrens (b) werden die Ausgangsstoffe der Formeln (VI), (VII), (IX) und (X) im allgemeinen in angenähert äquimolaren Mengen eingesetzt. Es ist jedoch auch möglich, eine der jeweils eingesetzten Komponenten in einem größeren Überschuß zu verwenden. Die Aufarbeitung erfolgt nach üblichen Methoden.

Die bei dem erfindungsgemäßen Verfahren (c) als Ausgangsstoffe benötigten Verbindungen sind durch die Formel (Ic) definiert. In dieser Formel steht $R^{16}$ für Chlor oder Brom. $R^{17}$ steht vorzugsweise für Chlor, Brom, Trifluor-

methyl oder Chlordifluormethyl. $R^{18}$ und $R^{19}$ stehen unabhängig voneinander vorzugsweise für Wasserstoff, Chlor, Brom, Trifluormethyl oder Chlordifluormethyl.X und Z haben vorzugsweise diejenigen Bedeutungen, die bereits im Zusammenhang mit der Beschreibung der erfindungsgemäßen Stoffe der Formel (I) vorzugsweise für diese Reste genannt wurden.

Die Verbindungen der Formel (Ic) sind bisher noch nicht bekannt. Sie lassen sich jedoch nach den erfindungsgemäßen Verfahren (a) und (b) herstellen.

Als Hydrierungskatalysatoren können bei dem erfindungsgemäßen Verfahren (c) alle üblicherweise für derartige Umsetzungen verwendbaren Katalysatoren eingesetzt werden. Vorzugsweise verwendbar sind Raney-Nickel oder Edelmetallkatalysatoren wie Palladium, Palladiumoxid, Platin oder Platinoxid, gegebenenfalls auf geeigneten Trägerstoffen, wie z.B. Kohle.

Als Verdünnungsmittel kommen bei der Durchführung des erfindungsgemäßen Verfahrens (c) alle unter Hydrierungsbedingungen inerten Solventien in Betracht. Vorzugsweise verwendbar sind Kohlenwasserstoffe, wie Pentan, Hexan, Heptan, Cyclohexan und Alkohole, wie Methanol, Ethanol, n-Propanol und iso-Propanol.

Die Temperaturen können auch bei der Durchführung des erfindungsgemäßen Verfahrens (c) innerhalb eines größeren Bereiches variiert werden. Im allgemeinen arbeitet man

bei Temperaturen zwischen 0°C und 100°C, vorzugsweise zwischen 20°C und 80°C.

Das erfindungsgemäße Verfahren (c) wird mit Wasserstoff unter erhöhtem Druck durchgeführt. Im allgemeinen arbeitet man bei Drucken zwischen 40 und 140 atm, vorzugsweise zwischen 60 und 120 atm.

Bei der Durchführung des erfindungsgemäßen Verfahrens (c) geht man im allgemeinen so vor, daß man die Verbindungen der Formel (Ic) in Gegenwart eines Katalysators und in Gegenwart eines Verdünnungsmittels bei der jeweils gewünschten Temperatur mit Wasserstoff im Autoklaven umsetzt. Die Aufarbeitung erfolgt nach üblichen Methoden.

Die bei dem erfindungsgemäßen Verfahren (d) als Ausgangsstoffe benötigten Verbindungen sind durch die Formel (Id) definiert. In dieser Formel haben $R^1$, $R^2$, $R^3$, $R^5$, $R^6$, X und Z vorzugsweise diejenigen Bedeutungen, die bereits im Zusammenhang mit der Beschreibung der erfindungsgemäßen Stoffe der Formel (I) vorzugsweise für diese Reste genannt wurden.

Die Verbindungen der Formel (Id) sind bisher noch nicht bekannt. Sie lassen sich jedoch nach den erfindungsgemäßen Verfahren (a), (b) und (c) herstellen.

Für die Nitrierung der Verbindungen der Formel (Id) verwendet man im Falle des erfindungsgemäßen Verfahrens (d) vorzugsweise konzentrierte Salpetersäure oder Schwermetallnitrate, wie zum Beispiel Eisen (III)-nitrat oder Kupfernitrat.

Als Katalysatoren kommen für die nach dem erfindungsgemäßen Verfahren (d) durchzuführende Nitrierung vorzugsweise Protonensäuren, wie zum Beispiel Schwefelsäure oder Essigsäure in Betracht.

Als Verdünnungsmittel kommen bei der Durchführung der Nitrierung nach dem erfindungsgemäßen Verfahren (d) vorzugsweise halogenierte Kohlenwasserstoffe, wie zum Beispiel Methylenchlorid oder 1,2-Dichlorethan, und ferner Carbonsäureanhydride, wie zum Beispiel Essigsäureanhydrid, in Frage.

Die Reaktionstemperaturen können bei der Nitrierung nach dem erfindungsgemäßen Verfahren (d) innerhalb eines größeren Bereiches variiert werden. Im allgemeinen arbeitet man bei Temperaturen zwischen -20°C und +60°C, vorzugsweise zwischen 0°C und +40°C.

Die Nitrierung nach dem erfindungsgemäßen Verfahren (d) wird im allgemeinen unter Normaldruck durchgeführt. Es ist jedoch auch möglich, unter erhöhtem oder vermindertem Druck zu arbeiten.

Bei der Durchführung der Nitrierung nach dem erfindungsgemäßen Verfahren (d) setzt man auf 1 Mol einer Verbindung der Formel (Id) im allgemeinen 1 bis 5 Mol, vorzugsweise 1,1 bis 3 Mol an Nitrierungsmittel und gegebenenfalls etwa die gleiche Menge eines Katalysators ein. Die Ausgangskomponenten werden vorzugsweise unter Eiskühlung zusammengegeben und dann gegebenenfalls bei leicht erhöhter Temperatur bis zum Ende der Umsetzung gerührt.

Die Aufarbeitung erfolgt nach üblichen Methoden. Im allgemeinen verfährt man in der Weise, daß man das Reaktionsgemisch nach beendeter Umsetzung in Eiswasser gießt, absaugt bzw. extrahiert und das anfallende Produkt gegebenenfalls durch Umkristallisieren reinigt.

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monocharia, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen z.B. Forst-, Ziergehölz-, Obst-, Wein-, Citrus-, Nuß-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Die erfindungsgemäßen Wirkstoffe können selektiv zur Bekämpfung von unerwünschtem Pflanzenwuchs in monokotylen Kulturen wie z.B. Getreide, Mais, Reis und Zuckerrohr eingesetzt werden.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, wirkstoffimprägnierte Natur- und synthetische Stoffe sowie Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glycol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:
z.B. Ammoniumsalze und natürliche Gesteinsmehle, wie Kaoline, Tonerden, Talkum, Kreide, Quarz, Attapulgit,

Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkolhol-Ether, z.B. Alkylaryl-polyglykolether, Alkylsulfonate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z.B. 1-Amino-6-ethylthio-3-(2,2-dimethylpropyl)-1,3,5-triazin-2,4(1H,3H)-dion oder N-(2-Benzthiazolyl)-N,N'-dimethylharnstoff zur Unkrautbekämpfung in Getreide; 4-Amino-3-methyl-6-phenyl-1,2,4-triazin-5(4H)-on zur Unkrautbekämfung in Zuckerrüben und 4-Amino-6-(1,1-dimethylethyl)-3-methylthio-1,2-4-triazin-5(4H)-on zur Unkrautbekämfung in Sojabohnen, in Frage. Einige Mischunngen zeigen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch insbesondere nach dem Auflaufen der Pflanzen appliziert werden.

Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 0,01 und 10 kg Wirkstoff pro Hektar Bodenfläche, vorzugsweise zwischen 0,05 und 5 kg pro ha.

Die Herstellung und die Verwendung der erfindungsgemäßen Wirkstoffe geht aus den nachfolgenden Beispielen hervor.

## Herstellungsbeispiele

### Beispiel 1

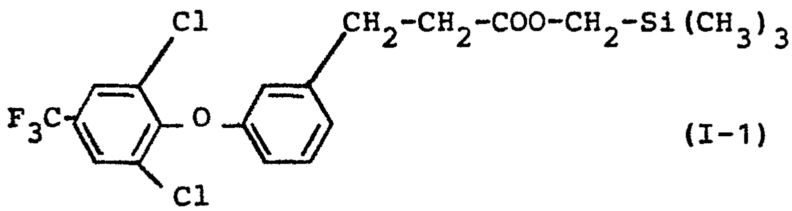

(I-1)

(Verfahren a , α)

7,2 g (0,019 Mol) 3-/3-(2,6-Dichlor-4-trifluormethyl-phenoxy)-phenyl/-propionsäure wurden in 50 ml Aceton gelöst und mit 3,5 g (0,022 Mol) 1,8-Diazabicyclo-(5.4.0)-undec-7-en versetzt. Dann wurden 2,7 g (0,022 Mol) Chlormethyltrimethylsilan zugetropft. Das Reaktionsgemisch wurde 18 1/2 Stunden unter Rückfluß gekocht, auf Raumtemperatur abgekühlt und filtriert. Das Filtrat wurde unter vermindertem Druck eingeengt. Den Rückstand reinigte man durch Chromatographie an einer Kieselgelsäule mit Methylenchlorid als Laufmittel.

Man erhielt 5,9 g (66,8 % der Theorie) an 3-/3-(2,6-Dichlor-4-trifluormethyl-phenoxy)-phenyl/-propionsäure-trimethylsilylmethylester.

$^{1}$H-NMR($CDCl_3$): $\delta$ = 2,67 (t, 2H, $-CH_2COO-$),
2,98 (t, 2H, Aryl-$CH_2$-),
3,81 (s, 2H, $-COO-CH_2-Si$) ppm.

Beispiel 2

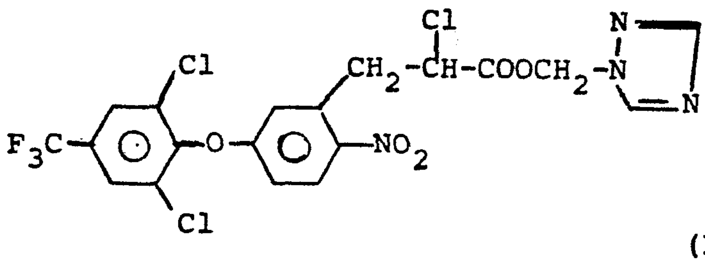

(I-2)

(Verfahren a, β)

3,2 g (0,007 Mol) 2-Chlor-3-/3-(2,6-dichlor-4-trifluormethyl-phenoxy)-6-nitro-phenyl7-propionsäure wurden in 40 ml Toluol gelöst, mit 1,3 ml Thionylchlorid und 0,1 g Triphenylphosphinoxid versetzt und 5 1/2 Stunden unter Rückfluß erhitzt. Das Toluol und der Überschuß an Thionylchlorid wurden unter vermindertem Druck abdestilliert. Das verbleibende Produkt wurde mit Toluol versetzt und bei 0°C bis 5°C zu einer Suspension von 0,7 g (0,007 Mol) 1-Hydroxymethyl-1,2,4-triazol und 1,2 ml Triethylamin in 20 ml Toluol getropft. Anschließend wurde das Reaktionsgemisch 16 Stunden bei 20°C gerührt, filtriert und unter vermindertem Druck eingeengt. Der Rückstand wurde mit Wasser verrührt, abgesaugt und bei 80°C getrocknet. Man erhielt 3,2 g (85 % der Theorie) an 2-Chlor-3-/3-(2,6-dichlor-4-trifluormethyl-phenoxy)-6-nitro-phenyl7-propionsäure-(1,2,4-triazol-1-yl)-methylester vom Schmelzpunkt 55-62°C.

Beispiel 3

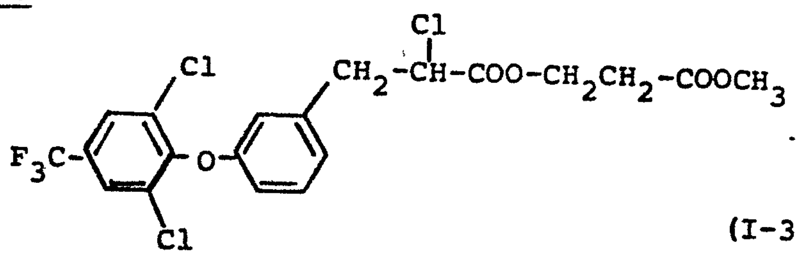

(I-3)

(Verfahren b)

32,2 g (0,1 Mol) 3-(2,6-Dichlor-4-trifluormethyl-phenoxy)-anilin wurden in einem Gemisch aus 170 ml Aceton und 45 ml konzentrierter Salzsäure bei 0°C bis 10°C mit 30 ml 30%iger wäßriger Natriumnitritlösung diazotiert. Daraufhin wurde das Diazoniumsalz mit 21 g Acrylsäure-(2-methoxy-carbonyl)-ethylester versetzt, und unter Rühren wurde eine Lösung von 1,5 g Kupfer(I)chlorid und 0,5 g Kupfer-II-chlorid in 25 ml halbkonzentrierter Salzsäure zugetropft. Das Reaktionsgemisch wurde 1 Stunde bei 20°C-25°C und eine weitere Stunde bei 40°C gerührt, mit Wasser versetzt und dann mit Ether extrahiert. Die Etherphase wurde mit 5%iger wäßriger Natriumhydrogencarbonatlösung gewaschen, über Natriumsulfat getrocknet, filtriert und eingeengt.

Man erhielt nach chromatographischer Reinigung über eine Kieselgelsäule mit Methylenchlorid als Laufmittel 21,4 g (43,6 % der Theorie) an 2-Chlor-3-/3-(2,6-dichlor-4-trifluormethyl-phenoxy)-phenyl/-propionsäure-2-(methoxycarbonyl)-ethylester.

$^{1}$H-NMR($CDCl_3$): $\delta$ = 2,61 (t, 1H, $-CH_2-CO_2-$), 3,12 (d, d, 1H, Aryl-$CH_2$-), 3,31 (d, d, 1H, Aryl-$CH_2$-), 4,38 (m, 3H, -CHCl-, -COO-$CH_2$-)ppm.

Beispiel 4

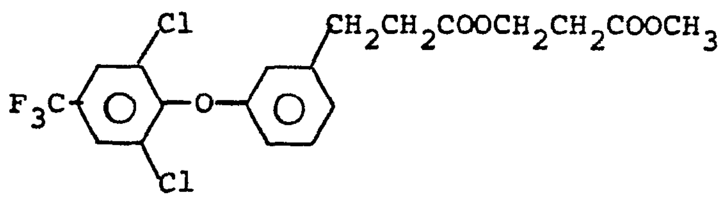

(I-4)

(Verfahren c)

5,2 g (0,0104 Mol) 2-Chlor-3-[3-(2,6-dichlor-4-trifluormethyl-phenoxy)-phenyl]-propionsäure-2-(methoxycarbonyl)-ethylester wurden in 50 ml Methanol gelöst und unter Zusatz von 0,7 g Raney-Nickel 2 Stunden bei 40°C unter einem Wasserstoff-Druck von 100 bar hydriert. Anschließend wurde der Katalysator abfiltriert und das Filtrat eingeengt.

Man erhielt 4,8 g (99 % der Theorie) an 3-[3-(2,6-Dichlor-4-trifluormethyl-phenoxy)-phenyl]-propionsäure-2-(methoxycarbonyl)-ethylester als gelbes Öl.

$^{1}$H-NMR($CDCl_3$): $\delta$ = 2,60 (m 4H, $-CH_2-COO-$), 2,90 (t, 2H, -Aryl-$CH_2$-), 3,68 (s, 3H, $-OCH_3$), 4,32 (t, 2H, $-COOCH_2-$) ppm.

Beispiel 5

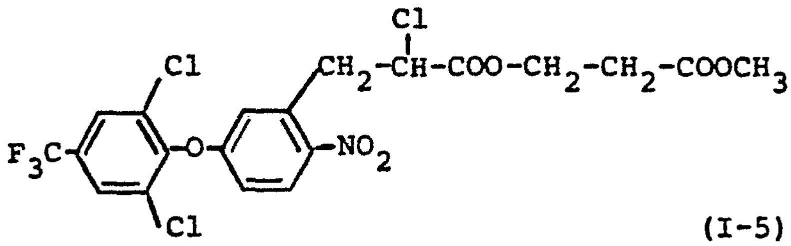

(I-5)

(Verfahren d)

10 g 2-Chlor-3-/3-(2,6-dichlor-4-trifluormethyl-phenoxy)-phenyl7-propionsäure-2-(methoxycarbonyl)-ethylester wurden in einem Gemisch aus 80 ml Eisessig und 20 ml Essigsäureanhydrid gelöst und bei 0°C bis 5°C unter Eiskühlung innerhalb von 20 Minuten mit 5 ml konzentrierter Salpetersäure versetzt. Das Reaktionsgemisch wurde anschließend 16 Stunden bei 20°C gerührt, mit 500 ml Eiswasser versetzt und mit 300 ml Methylenchlorid extrahiert. Die wäßrige Phase wurde noch zweimal mit jeweils 100 ml Methylenchlorid extrahiert. Die vereinigten organischen Phasen wurden mit 5-%iger wäßriger Natriumcarbonat-Lösung gewaschen, über Natriumcarbonat getrocknet, filtriert und eingeengt. Man erhielt 10,9 g (100 % der Theorie) an 2-Chlor-3-/3-(2,6-dichlor-4-trifluormethyl-phenoxy)-6-nitro-phenyl7-propionsäure-2-(methoxycarbonyl)-ethylester.

$^1H$-NMR($CDCl_3$): $\delta$ = 2,66 (t, 1H, $-CH_2-CO_2-$), 3,34 (d, d, 1H, Aryl-$CH_2$-), 3,68 (m, 4H, $-OCH_3$, Aryl-$CH_2$-), 4,41 (t, 1H, $-COOCH_2-$), 4,64 (d, d, 1H, -CHCl-) ppm.

Analog den Beispielen 1 bis 5 bzw. Verfahren (a) bis (d) wurden die folgenden Verbindungen der Formel (I) hergestellt:

Beispiel 6

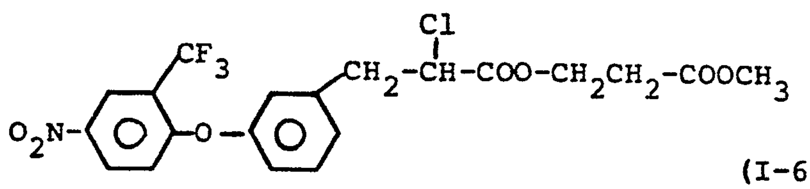

(I-6)

$^{1}H$-NMR($CDCl_3$): $\delta$ = 2,65 (t, 2H, $-CH_2-CO_2-$), 3,20 (d, d, 1H, Aryl-$CH_2$-), 3,38 (d, d, 1H, Aryl-$CH_2$-), 4,42 (m, 3H, -CHCl-; $-COOCH_2-$) ppm.

Beispiel 7

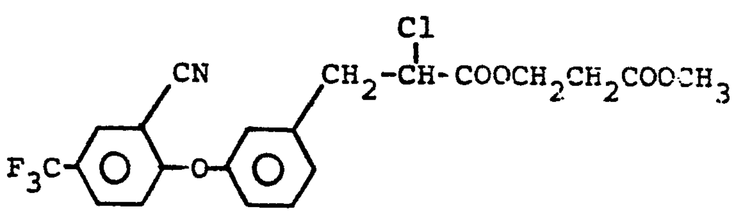

(I-7)

$^{1}H$-NMR($CDCl_3$): $\delta$ = 2,64 (t, 2H, $-CH_2-CO_2-$), 3,19 (d, d, 1H, Aryl-$CH_2$-), 3,37 (d, d, 1H, Aryl-$CH_2$-), 4,42 (m, 3H, -CHCl-, $-COOCH_2-$) ppm

Beispiel 8

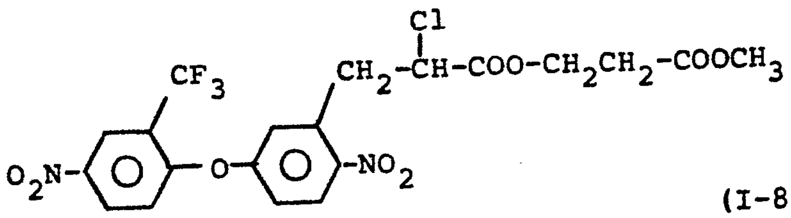

(I-8)

$^1H$-NMR($CDCl_3$): $\delta$ = 2,70 (t, 2H, $-CH_2-CO_2-$), 3,40 (d, d, 1H, Aryl-$CH_2$-), 3,75 (m, 4H, Aryl-$CH_2$, $-COOCH_3$), 4,44 (t, 2H, $-COOCH_2-$) 4,70 (d, d, 1H, -CHCl-) ppm.

Beispiel 9

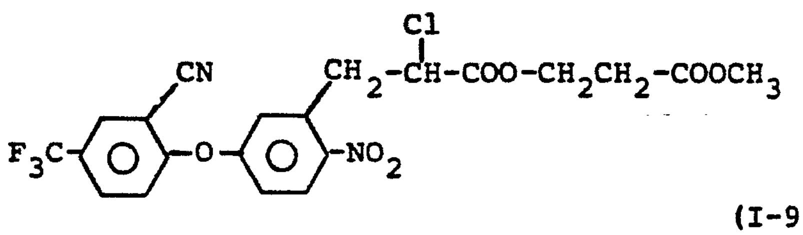

(I-9)

$^1H$-NMR($CDCl_3$): $\delta$ = 2,70 (t, 2H, $-CH_2-CO_2-$), 3,38 (m, 1H, Aryl-$CH_2$-), 3,69 (m, 4H, Aryl-$CH_2$-, $-COOCH_3$), 4,44 (t, 2H, $-COOCH_2-$), 4,70 (d, d, 1H, -CHCl-) ppm.

Beispiel 10

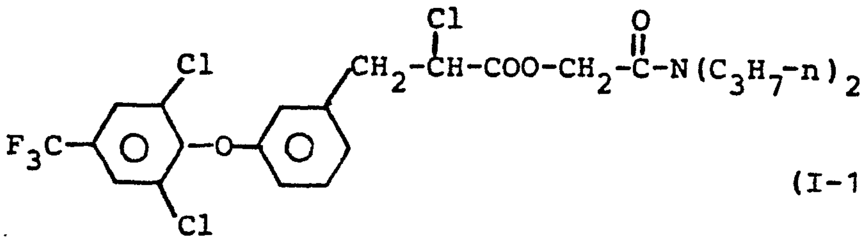

(I-10)

$^1$H-NMR($CDCl_3$): $\delta$ = 3,0-3,54 (m, 6H, $-N-CH_2$, $-Aryl-CH_2$), 4,54 (d, d, 1H, -CHCl-), 4,74 (d, 1H, $-COOCH_2-CO-$), 4,84 (d, 1H, $-COOCH_2-CO$) ppm.

Beispiel 11

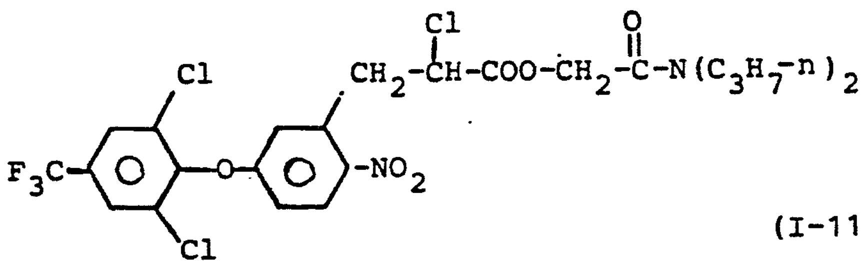

(I-11)

$^1$H-NMR($CDCl_3$): $\delta$ = 3,o6-3,42 (m, 5H, $-N-CH_2-$, $Aryl-CH_2-$), 3,98 (d, d, 1H, $Aryl-CH_2-$), 4,82 (m, 3H, -CHCl-; $-COOCH_2-CO-$) ppm.

Beispiel 12

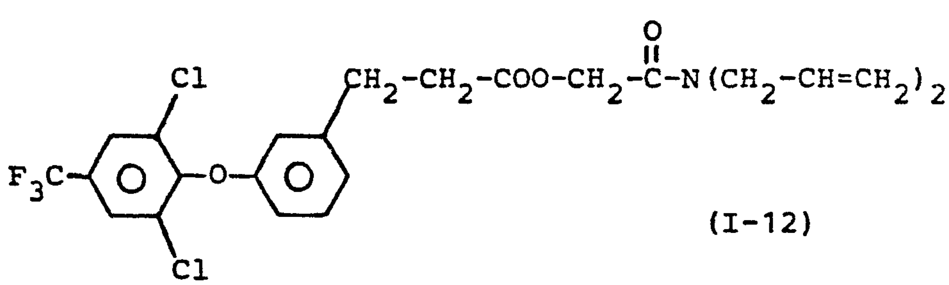

(I-12)

$^{1}H$-NMR($CDCl_3$): $\delta$ = 2,74 (t, 2H, $-CH_2-COO-$), 2,98 (t, 2H, Aryl-$CH_2$-), 3,78 (d, d, 2H, $-N-CH_2$), 4,04 (d, d, 2H, $-N-CH_2-$), 4,73 (s, 2H, $-COOCH_2-$) ppm.

<u>Beispiel 13</u>

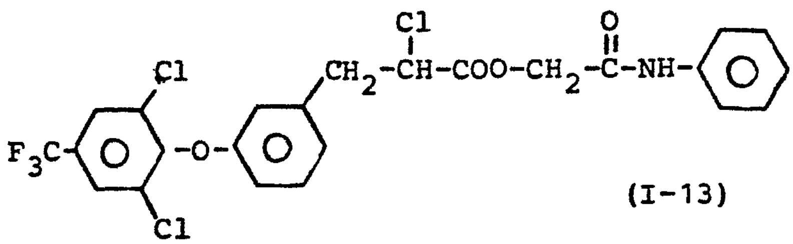

(I-13)

$F_p$: 48-53°C

<u>Beispiel 14</u>

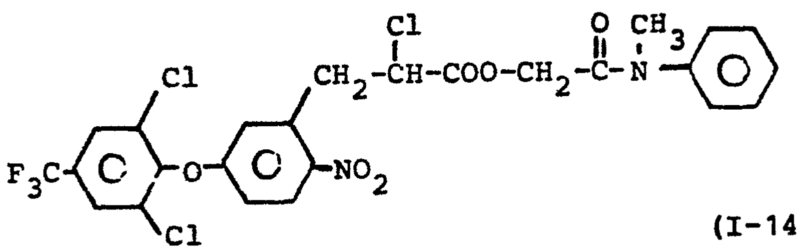

(I-14)

$^{1}H$-NMR($CDCl_3$): $\delta$ = 3,34 (m, 4H, $-N-CH_3$, Aryl-$CH_2$-), 3,92 (d, d, 1H, Aryl-$CH_2$-), 4,57 (s, $-COO-CH_2-CO-$), 4,78 (d, d, 1H, -CHCl-) ppm.

<u>Beispiel 15</u>

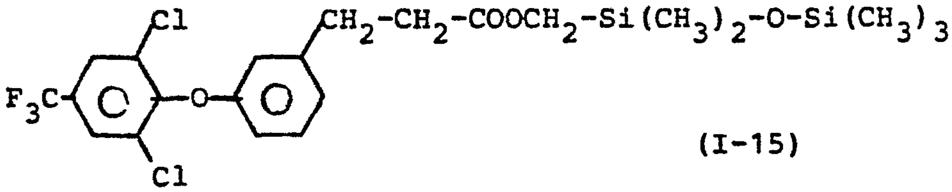

(I-15)

$^1H$-NMR($CDCl_3$): $\delta$ = 2,6 (t, 2H, $-CH_2-COO-$), 2,91 (t, 2H, Aryl-$CH_2$-), 3,70 (s, 2H, $-COO-CH_3-$) ppm.

Beispiel 16

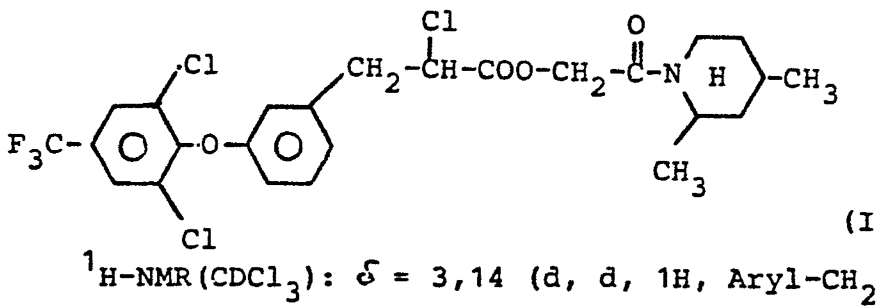

(I-16)

$^1H$-NMR($CDCl_3$): $\delta$ = 3,14 (d, d, 1H, Aryl-$CH_2$-), 3,48 (d, d, 1H, Aryl-$CH_2$-), 4,52 (d, d, 1H, -CHCl-),4,70 (d, 1H, -COO-$CH_2$-), 4,79 (d, 1H, $-COOCH_2-$) ppm.

Beispiel 17

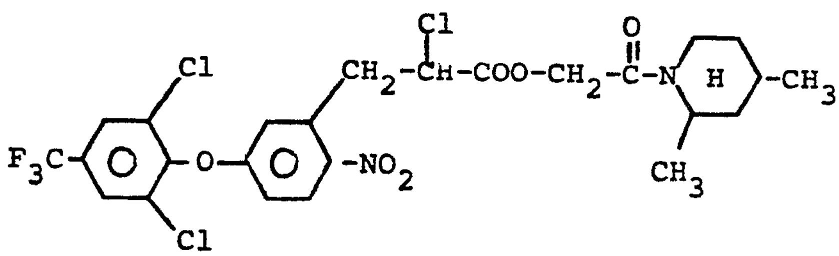

(I-17)

Zersetzungsp.: 145°C

Beispiel 18

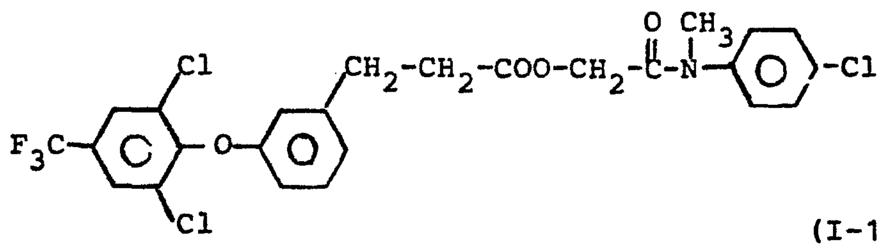

(I-18)

$^1H$-NMR($CDCl_3$): $\delta$ = 2,69 (t, 2H, $-CH_2-COO-$), 2,93 (t, 2H, Aryl-$CH_2-$), 3,25 (s, 3H, $-\overset{|}{N}-CH_3$), 4,36 (s, 2H, $-COOCH_2-$) ppm.

Beispiel 19

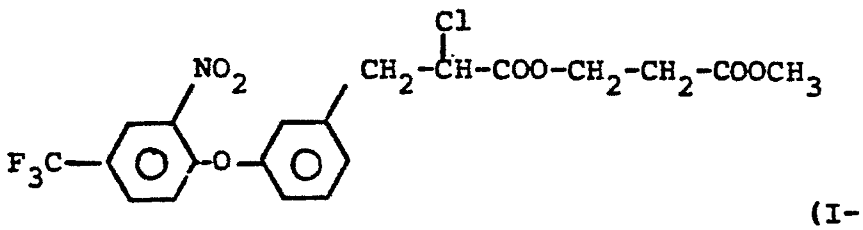

(I-19)

$^1H$-NMR($CDCl_3$): $\delta$ = 2,64 (t, 2H, $-CH_2COO-$), 3,19 (d, d, 1H, Aryl-$CH_2-$), 3,36 (d, d, 1H, Aryl-$CH_2-$), 3,66 (s, 3H, $-COOCH_3$), 4,40 (m, 3H, $-COOCH_2-$; $-CHCl-$) ppm.

Beispiel 20

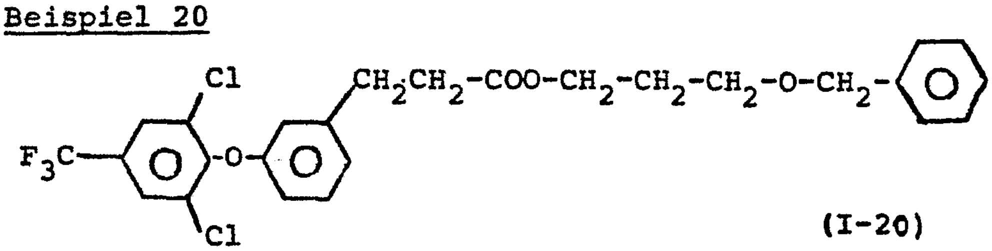

(I-20)

$^1$H-NMR(CDCl$_3$): δ = 1,9 (m, 2H, $-\overset{|}{\underset{|}{C}}-CH_2-\overset{|}{\underset{|}{C}}-$), 2,56 (t, 2H, $-CH_2-COO-$), 2,89 (t, 2H, Aryl-$CH_2-\overset{|}{\underset{|}{C}}-$), 3,49 (t, 2H, $-\overset{|}{\underset{|}{C}}-CH_2-O-$), 4,16 (t, 2H, $-COO-CH_2-$), 4,46 (s, 2H, $-O-CH_2-$Aryl) ppm.

Beispiel 21

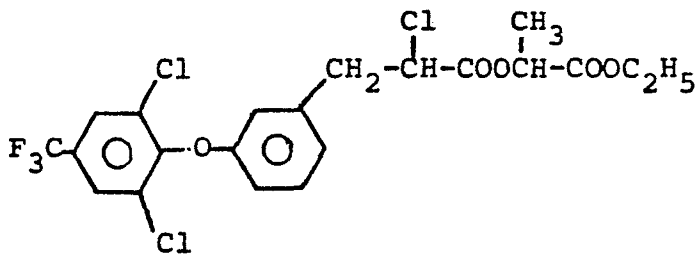

(I-21)

$n_D^{20}$ = 1,5253

Beispiel 22

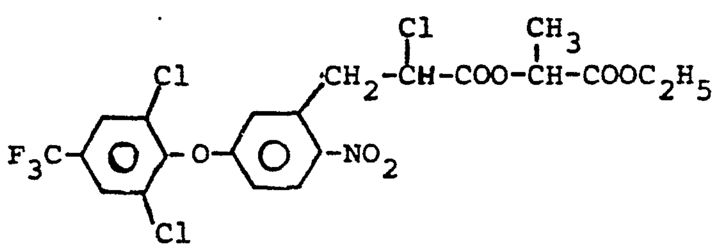

(I-22)

$n_D^{20}$ = 1,5371

Beispiel 23

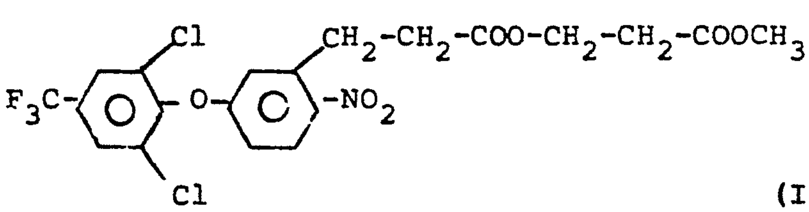

(I-23)

$^{1}H$-NMR($CDCl_3$): $\delta$ = 2,62 (t, 2H, $-CH_2-COO-$), 2,69 (t, 2H, $-CH_2-COO-$), 3,22 (t, 2H, Aryl-$CH_2-$), 3,67 (s, 3H, $-COO-CH_3$), 4,31 (t, 2H, $-COO-CH_2-$) ppm.

Beispiel 24

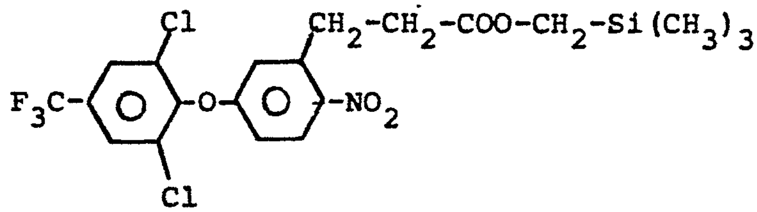

(I-24)

$^{1}H$-NMR($CDCl_3$): $\delta$ = 2,72 (t, 2H, $-CH_2-COO-$), 3,26 (t, 2H, Aryl-$CH_2-$), 3,77 (s, 2H, $-COOCH_2-\overset{|}{\underset{|}{Si}}-$) ppm

Beispiel 25

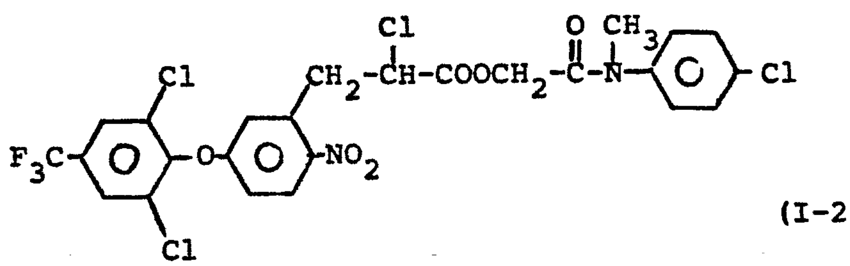

(I-25)

$^{1}H$-NMR($CDCl_3$): $\delta$ = 3,20-3,40 (m, 4H, $-N-CH_3$; Aryl-$CH_2-$), 3,91 (d, d, 1H, Aryl-$CH_2-$), 4,45 (m, 2H, $-COOCH_2-$), 4,76 (d, d, 1H, $-CHCl-$) ppm.

Beispiel 26

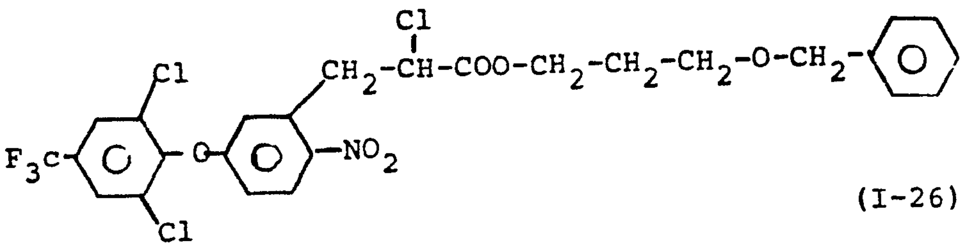

(I-26)

$^1$H-NMR($CDCl_3$): = 1,94 (m, 2H, $-C-CH_2-C-$), 3,30 (d, d, 1H, Aryl-$CH_2$-), 3,50 (t, 2H, $-C-CH_2-O-$), 3,70 (d, d, 1H, Aryl-$CH_2$-), 4,26 (m, 2H, $-COOCH_2-$), 4,46 (s, 2H, $-O-CH_2$-Aryl), 4,61 (d, d, 1H, -CHCl-) ppm.

Herstellung von Ausgangsprodukten

Beispiel 27

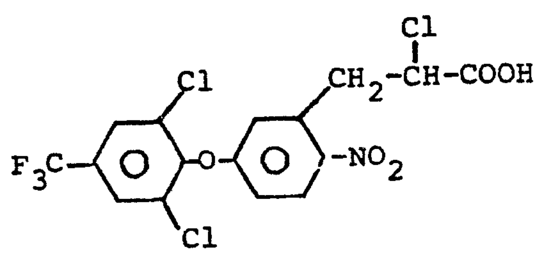

(II-1)

30 g 2-Chlor-3-[3-(2,6-dichlor-4-trifluormethyl-phenoxy)-6-nitro-phenyl]-propionsäuremethylester wurden in 250 ml Ameisensäure gelöst und 15 Stunden unter Rückfluß erhitzt, wobei ein Salzsäuregasstrom durch das Reaktionsgemisch geleitet wurde. Das Reaktionsgemisch wurde anschließend unter vermindertem Druck eingeengt. Der Rückstand wurde in 500 ml Methylenchlorid aufgenommen und mehrmals mit Wasser gewaschen. Die organische Phase wurde über Natriumsulfat getrocknet, filtriert und eingeengt. Das Reaktionsprodukt wurde mit n-Hexan zur Kristallisation gebracht. Man erhielt 18,6 g (64 % der Theorie) an 2-Chlor-3-/3-(2,6-dichlor-4-trifluormethyl-phenoxy)-6-nitro-phenyl7-propionsäure vom Schmelzpunkt 143°C-146°C.

Beispiel 28

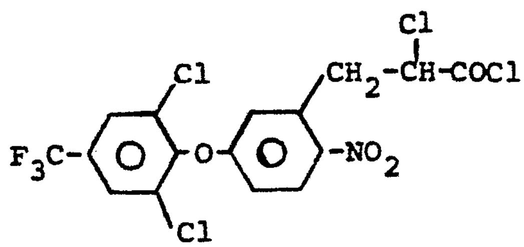

(IV-1)

16,0 g (0,0366 Mol) 2-Chlor-3-/3-(2,6-dichlor-4-trifluormethyl-phenoxy)-6-nitro-phenyl7-propionsäure wurden in 200 ml Toluol gelöst mit 10,8 g Thionylchlorid und 0,1 g Triphenylphosphinoxid versetzt und 5 Stunden unter Rückfluß erhitzt. Nach Beendigung der Gasentwicklung wurde das Lösungsmittel mit dem überschüssigen Thionylchlorid unter vermindertem Druck abdestilliert. Man erhielt 16,3 g (98 % der Theorie) an 2-Chlor-3-/3-(2,6-dichlor-4-trifluormethyl-phenoxy)-6-nitro-phenyl7-propionsäurechlorid.

Beispiel 29

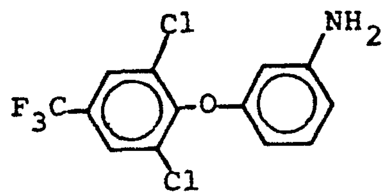

(VI-1)

65 g 3-Aminophenol wurden mit 28 g Natriumhydroxid in 500 ml Dimethylsulfoxid 30 Minuten bei 120°C gerührt. Anschließend wurden 150 g 3,4,5-Trichlor-1-trifluormethyl-benzol zugetropft, und es wurde 5 Stunden bei 120°C gerührt. Nach dem Abkühlen auf 25°C wurde das Reaktionsgemisch in 1 l Wasser eingerührt und mit Methylenchlorid extrahiert. Die organische Phase wurde getrocknet, filtriert und eingeengt. Man erhielt 200 g rohes 3-(2,6-Dichlor-4-trifluormethyl-phenoxy)-anilin. Zur Reinigung wurde das Produkt in 100 ml Methanol eingerührt. Dann wurden 400 ml konzentrierte Salzsäure und 200 ml Wasser zugegeben, es wurde abgesaugt und bei 30°C im Vakuum getrocknet. Auf diese Weise wurden 192 g des Hydrochlorids von 3-(2,6-Dichlor-4-trifluormethyl-phenoxy)-anilin erhalten. Schmelzpunkt 200-204°C.

Verwendungsbeispiele

In dem nachstehend beschriebenen Verwendungsbeispiel wurde folgende Verbindung als Vergleichssubstanz eingesetzt:

(A) = 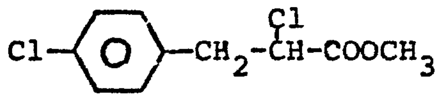

2-Chlor-3-(4-chlorphenyl)-propionsäure-methylester

(bekannt aus GB-PS 1 077 194)

Beispiel A

Post-emergence-Test/Gewächshaus

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben so, daß die jeweils gewünschten Wirkstoffmengen pro Flächeneinheit ausgebracht werden. Die Konzentration der Spritzbrühe wird so gewählt, daß in 2 000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle.
Es bedeuten:

0 % = keine Wirkung (wie unbehandelte Kontrolle)
100 % = totale Vernichtung

In diesem Test zeigten die erfindungsgemäßen Wirkstoffe (I-2), (I-5), (I-22), (I-23) und (II-1), bei der Bekämpfung von Galinsoga, Matricaria, Sinapis, Stellaria, Galium, Avena fatua und Portulaca in Weizen eine bessere selektive herbizide Wirksamkeit als die Vergleichssubstanz (A).

Patentansprüche

1. Substituierte Diphenylether und Diphenylthioether der Formel

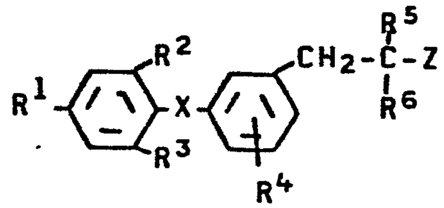

(I)

in welcher

X für Sauerstoff oder Schwefel steht,

$R^1$ für Halogen, Trihalogenmethyl, Trihalogenmethylsulfonyl, Cyano oder Nitro steht,

$R^2$ und $R^3$ unabhängig voneinander für Wasserstoff, Halogen, Trihalogenmethyl, Trihalogenmethylsulfonyl, Nitro oder Cyano stehen,

$R^4$ für Wasserstoff, Nitro oder Cyano steht,

$R^5$ und $R^6$ unabhängig voneinander für Wasserstoff, Chlor oder Brom stehen und

Z für Formyl oder den Rest der Formel

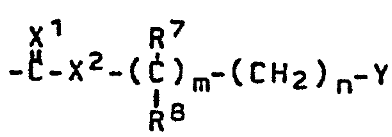

steht,

in welcher

$X^1$ und $X^2$ unabhängig voneinander für Sauerstoff oder Schwefel stehen,

$R^7$ und $R^8$ unabhängig voneinander für Wasserstoff oder Alkyl mit 1 bis 4 Kohlenstoffatomen stehen,

m für 0, 1 oder 2 steht,

n für 0 oder 1 steht und

Y für Cyano, Alkoxycarbonyl, gegebenenfalls substituiertes über Stickstoff gebundenes Azolyl, gegebenenfalls substituiertes Cycloalkyl oder Cycloalkenyl sowie für die Reste der Formeln

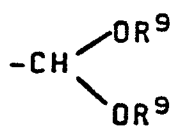 ; $-Si(CH_3)_2R^{10}$ ; 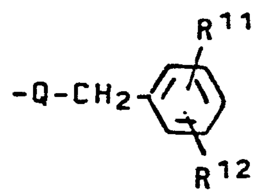 oder

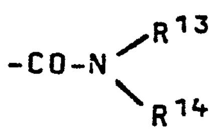 steht,

in welchen

$R^9$ für Alkyl mit 1 bis 4 Kohlenstoffatomen steht oder die beiden Reste $R^9$ gemeinsam für eine Alkylenkette mit 2 oder 3 Kohlenstoffatomen stehen,

$R^{10}$ für Alkyl mit 1 bis 4 Kohlenstoffatomen oder für Trimethylsilyloxy steht,

Q für Sauerstoff, Schwefel, SO oder $SO_2$ steht,

$R^{11}$ und $R^{12}$ unabhängig voneinander für Wasserstoff, Halogen, Alkyl mit 1 bis 4 Kohlenstoffatomen, Alkoxy mit 1 bis 4 Kohlenstoffatomen, Alkyl thio mit 1 bis 4 Kohlenstoffatomen, Nitro, Cyano oder Alkoxycarbonyl mit 1 bis 4 Kohlenstoffatomen in der Alkoxygruppe stehen und

$R^{13}$ und $R^{14}$ unabhängig voneinander für Wasserstoff, für Alkyl mit 1 bis 6 Kohlenstoffatomen, für Alkenyl und Alkinyl mit jeweils 2 bis 6 Kohlenstoffatomen, für gegebenenfalls ein- bis dreifach, gleichartig oder verschieden durch Methyl oder Ethyl substituiertes Cycloalkyl oder Cycloalkenyl mit 5 bis 7 Kohlenstoffatomen, für Alkoxy mit 1 bis 6 Kohlenstoffatomen, Alkoxyalkylenoxy mit 1 bis 6 Kohlenstoffatomen in der Alkoxygruppe und 1 bis 6 Kohlenstoffatomen im Alkylenoxy-Teil, Alkoxyalkyl mit 1 bis 6 Kohlenstoffatomen in der Alkoxygruppe und 1 bis 6 Kohlenstoffatomen im Alkyl-Teil, für Halogenalkyl mit 1 bis 6 Kohlenstoffatomen und 1 bis 5 Halogenatomen, für Benzyl sowie für gegebenenfalls einfach bis dreifach, gleichartig oder verschieden durch Methyl, Ethyl, Methoxy, Methylthio, Trifluormethyl, Trifluormethoxy, Trifluormethylthio, Fluor, Chlor und/oder Nitro substituiertes Phenyl stehen oder

$R^{13}$ und $R^{14}$ zusammen mit dem Stickstoffatom, an das sie gebunden sind, für gegebenenfalls substituiertes Piperidinyl, gegebenenfalls substituiertes Pyrrolidinyl, gegebenenfalls substituiertes Hexahydroazepinyl, gegebenenfalls substituiertes Morpholinyl, gegebenenfalls substituiertes Tetrahydrochinolyl oder gegebenenfalls substituiertes Perhydrochinolyl stehen.

2. Substituierte Diphenylether und Diphenylthioether der Formel (I), in denen

X für Sauerstoff oder Schwefel steht,

$R^1$ für Chlor, Brom, Trifluormethyl, Chlordifluormethyl, Trifluormethylsulfonyl, Cyano oder Nitro steht,

$R^2$ und $R^3$ unabhängig voneinander für Wasserstoff, Chlor, Brom, Trifluormethyl, Chlordifluormethyl, Trifluormethylsulfonyl, Nitro oder Cyano stehen,

$R^4$ für Wasserstoff, Nitro oder Cyano steht,

$R^5$ und $R^6$ unabhängig voneinander für Wasserstoff, Chlor oder Brom stehen und

Z für Formyl oder den Rest der Formel

$$-\overset{X^1}{\overset{\|}{C}}-X^2-(\underset{R^8}{\underset{|}{\overset{R^7}{\overset{|}{C}}}})_m-(CH_2)_n-Y$$

steht

worin

$X^1$ und $X^2$ unabhängig voneinander für Sauerstoff oder Schwefel stehen,

$R^7$ und $R^8$ unabhängig voneinander für Wasserstoff oder Methyl stehen,

m für 0, 1 oder 2 steht,

n für 0 oder 1 steht und

Y für Cyano, Alkoxycarbonyl mit 1 bis 4 Kohlenstoffatomen im Alkoxyteil oder für einen über ein Ring-Stickstoffatom gebundenen Pyrazoyl-, Imidazolyl-, 1,2,4,-Triazolyl-, 1,2,3-Triazolyl- oder 1,3,4-Triazolyl-Rest steht, wobei jeder dieser Azolyl-Reste ein- oder mehrfach, gleichartig oder verschieden durch Fluor, Chlor, Brom, Jod, Alkyl mit 1 bis 4 Kohlenstoffatomen, Alkoxy mit 1 bis 4 Kohlenstoffatomen und/oder Phenyl substituiert sein kann,

und

Y weiterhin für gegebenenfalls ein- oder mehrfach, gleichartig oder verschieden durch Alkyl mit 1 bis 4 Kohlenstoffatomen substituiertes Cycloalkyl mit 3 bis 8 Kohlenstoffatomen im Cycloalkylteil, für gegebenenfalls ein- oder mehrfach, gleichartig oder verschieden durch Alkyl mit 1 bis 4 Kohlenstoffatomen substituiertes Cycloalkenyl mit 5 bis 8 Kohlenstoffatomen im Cycloalkenylteil oder für die Reste der Formeln

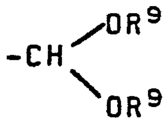 ; $-Si(CH_3)_2R^{10}$ ; 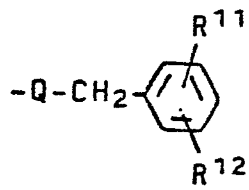 oder

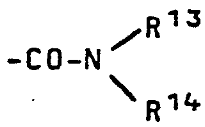 steht,

in welchen

$R^9$ für Alkyl mit 1 oder 2 Kohlenstoffatomen steht, oder die beiden Reste $R^9$ gemeinsam für eine Alkylenkette mit 2 oder 3 Kohlenstoffatomen stehen,

$R^{10}$ für Alkyl mit 1 oder 2 Kohlenstoffatomen oder für Trimethylsilyloxy steht,

Q für Sauerstoff, Schwefel, SO oder $SO_2$ steht,

$R^{11}$ und $R^{12}$ unabhängig voneinander für Wasserstoff, Fluor, Chlor, Brom, Jod, Alkyl mit 1 oder 2 Kohlenstoffatomen, Alkoxy mit 1 oder 2 Kohlenstoffatomen Alkylthio mit 1 oder 2 Kohlenstoffatomen, Nitro, Cyano oder Alkoxycarbonyl mit 1 bis 2 Kohlenstoffatomen in der Alkoxygruppe stehen,

und

$R^{13}$ und $R^{14}$ unabhängig voneinander für Wasserstoff, Alkyl mit 1 bis 4 Kohlenstoffatomen, Alkenyl und Alkinyl mit jeweils 2 bis 4 Kohlenstoffatomen, für gegebenenfalls ein- oder zweifach, gleichartig oder verschieden durch Methyl oder Ethyl substituiertes Cycloalkyl mit 5 bis 7 Kohlenstoffatomen, für gegebenenfalls ein- oder zweifach, gleichartig oder verschieden durch Methyl oder Ethyl substituiertes Cycloalkenyl mit 5 bis 7 Kohlenstoffatomen, für Alkoxy mit 1 bis 4 Kohlenstoffatomen, Alkoxyalkylenoxy mit 1 bis 4 Kohlenstoffatomen in der Alkoxygruppe und 1 bis 4 Kohlenstoffatomen im Alkylenoxy-Teil, Alkoxyalkyl mit 1 bis 4 Kohlenstoffatomen in der Alkoxygruppe und 1 bis 4 Kohlenstoffatomen im Alkylteil, für Halogenalkyl mit 1 bis 4 Kohlenstoffatomen und 1 bis 5 Fluor-, Chlor- und/oder Brom-Atomen, für Benzyl sowie für gegebenenfalls ein- bis dreifach, gleichartig oder verschieden durch Methyl, Ethyl, Methoxy, Methylthio, Trifluormethyl, Trifluormethoxy, Trifluormethylthio, Fluor, Chlor und/oder Nitro substituiertes Phenyl stehen,

oder

$R^{13}$ und $R^{14}$ zusammen mit dem Stickstoffatom, an das sie gebunden sind, für Piperidinyl, Pyrrolidinyl, Hexahydroazepinyl, Morpholinyl, Tetrahydrochinolyl oder Perhydrochinolyl stehen, wobei jeder dieser heterocyclischen Reste einfach bis dreifach, gleichartig oder verschieden durch Methyl und/oder Ethyl substituiert sein kann.

3. Verfahren zur Herstellung von substituierten Diphenylethern und Diphenylthioethern der Formel

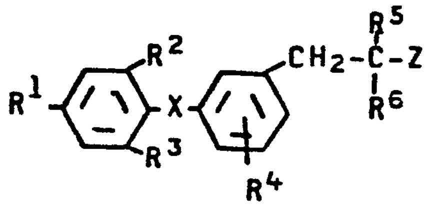

(I)

in welcher

X für Sauerstoff oder Schwefel steht,

$R^1$ für Halogen, Trihalogenmethyl, Trihalogenmethylsulfonyl, Cyano oder Nitro steht,

$R^2$ und $R^3$ unabhängig voneinander für Wasserstoff, Halogen, Trihalogenmethyl, Trihalogenmethylsulfonyl, Nitro oder Cyano stehen,

$R^4$ für Wasserstoff, Nitro oder Cyano steht,

$R^5$ und $R^6$ unabhängig voneinander für Wasserstoff, Chlor oder Brom stehen und

Z für Formyl oder den Rest der Formel

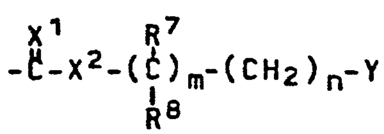

steht,

in welcher

$X^1$ und $X^2$ unabhängig voneinander für Sauerstoff oder Schwefel stehen,

$R^7$ und $R^8$ unabhängig voneinander für Wasserstoff oder Alkyl mit 1 bis 4 Kohlenstoffatomen stehen,

m für 0, 1 oder 2 steht,

n für 0 oder 1 steht und

Y für Cyano, Alkoxycarbonyl, gegebenenfalls substituiertes über Stickstoff gebundenes Azolyl, gegebenenfalls substituiertes Cycloalkyl oder Cycloalkenyl sowie für die Reste der Formeln

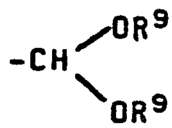 ; $-Si(CH_3)_2R^{10}$ ; 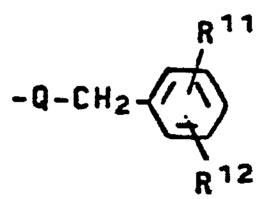 oder

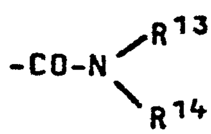 steht,

in welchen

$R^9$ für Alkyl mit 1 bis 4 Kohlenstoffatomen steht oder die beiden Reste $R^9$ gemeinsam für eine

Alkylenkette mit 2 oder 3 Kohlenstoffatomen stehen,

$R^{10}$ für Alkyl mit 1 bis 4 Kohlenstoffatomen oder für Trimethylsilyloxy steht,

Q für Sauerstoff, Schwefel, SO oder $SO_2$ steht,

$R^{11}$ und $R^{12}$ unabhängig voneinander für Wasserstoff, Halogen, Alkyl mit 1 bis 4 Kohlenstoffatomen, Alkoxy mit 1 bis 4 Kohlenstoffatomen, Alkylthio mit 1 bis 4 Kohlenstoffatomen, Nitro, Cyano oder Alkoxycarbonyl mit 1 bis 4 Kohlenstoffatomen in der Alkoxygruppe stehen und

$R^{13}$ und $R^{14}$ unabhängig voneinander für Wasserstoff, für Alkyl mit 1 bis 6 Kohlenstoffatomen, für Alkenyl und Alkinyl mit jeweils 2 bis 6 Kohlenstoffatomen, für gegebenenfalls ein- bis dreifach, gleichartig oder verschieden durch Methyl oder Ethyl substituiertes Cycloalkyl oder Cycloalkenyl mit 5 bis 7 Kohlenstoffatomen, für Alkoxy mit 1 bis 6 Kohlenstoffatomen, Alkoxyalkylenoxy mit 1 bis 6 Kohlenstoffatomen in der Alkoxygruppe und 1 bis 6 Kohlenstoffatomen im Alkylenoxy-Teil, Alkoxyalkyl mit 1 bis 6 Kohlenstoffatomen in der Alkoxygruppe und 1 bis 6 Kohlenstoffatomen im Alkyl-Teil, für Halogenalkyl mit 1 bis 6 Kohlenstoffatomen und 1 bis 5 Halogenatomen, für Benzyl sowie für gegebenenfalls einfach bis dreifach, gleichartig oder verschieden durch Methyl, Ethyl, Methoxy, Methylthio, Trifluormethyl, Trifluormethoxy, Trifluormethylthio, Fluor, Chlor und/oder Nitro substituiertes Phenyl stehen oder

$R^{13}$ und $R^{14}$ zusammen mit dem Stickstoffatom, an das sie gebunden sind, für gegebenenfalls substituiertes Piperidinyl, gegebenenfalls substituiertes Pyrrolidinyl, gegebenenfalls substituiertes Hexahydroazepinyl, gegebenenfalls substituiertes Morpholinyl, gegebenenfalls substituiertes Tetrahydrochinolyl oder gegebenenfalls substituiertes Perhydrochinolyl stehen,

dadurch gekennzeichnet, daß man

a) zur Herstellung derjenigen substituierten Diphenylether und Diphenylthioether der Formel (I), in denen

Z für den Rest der Formel

$$-\overset{\overset{\displaystyle X^1}{\|}}{C}-X^2-(\underset{\underset{\displaystyle R^8}{|}}{\overset{\overset{\displaystyle R^7}{|}}{C}})_m-(CH_2)_n-Y$$ steht,

worin

$X^1$, $X^2$, Y, $R^7$, $R^8$, m und n die oben angegebenen Bedeutungen haben,

so vorgeht, daß man

α) (Thio)Carbonsäure-Derivate der Formel

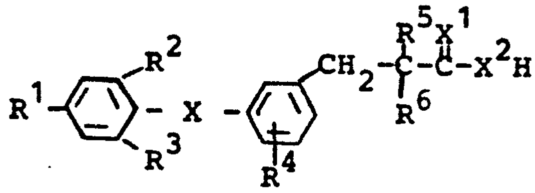

(II)

in welcher

$R^1$, $R^2$, $R^3$, $R^4$, $R^5$, $R^6$, X, $X^1$ und $X^2$ die oben angegebenen Bedeutungen haben,

mit Verbindungen der Formel

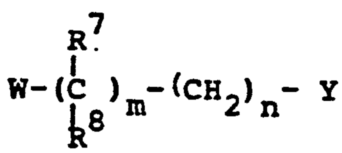

(III)

in welcher

Y, $R^7$, $R^8$, m und n die oben angegebenen Bedeutungen haben und

W für Chlor, Brom, Mesylat oder Tosylat steht,

gegebenenfalls in Gegenwart eines Säurebindemittels sowie gegebenenfalls in Gegenwart eines Verdünnungsmittels umsetzt,

oder

β) (Thio)Carbonsäurechloride der Formel

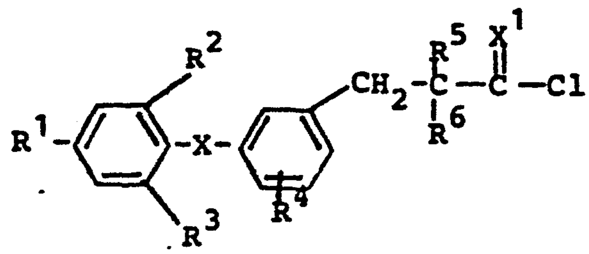

(IV)

in welcher

$R^1$, $R^2$, $R^3$, $R^4$, $R^5$, $R^6$, X und $X^1$ die oben angegebenen Bedeutungen haben,

mit Verbindungen der Formel

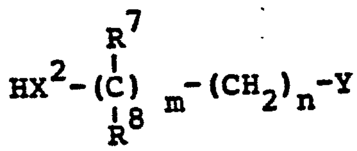

(V)

in welcher

$X^2$, Y, $R^7$, $R^8$, m und n die oben angegebenen Bedeutungen haben,

gegebenenfalls in Gegenwart eines Säurebindemittels sowie gegebenenfalls in Gegenwart eines Verdünnungsmittels umsetzt,

b) zur Herstellung von Verbindungen der Formel

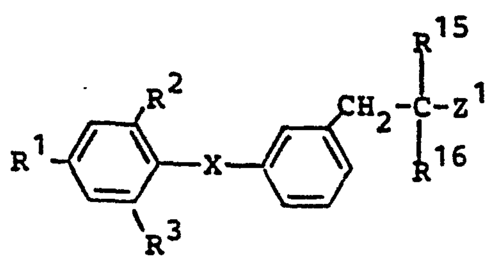

(Ia)

in welcher

$R^1$, $R^2$, $R^3$ und X die oben angegebenen Bedeutungen haben,

$R^{15}$ für Wasserstoff, Chlor oder Brom steht,

$R^{16}$ für Chlor oder Brom steht und

$Z^1$ für Formyl oder den Rest der Formel

$$-\overset{O}{\overset{\|}{C}}-O-CH_2-CH_2-Y^1$$ steht,

worin

$Y^1$ für Alkoxycarbonyl steht,

so vorgeht, daß man Aniline der Formel

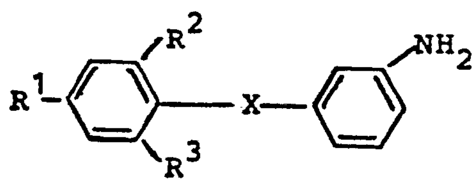

(VI)

in welcher

$R^1$, $R^2$, $R^3$ und X die oben angegebenen Bedeutungen haben,

oder Säureadditionssalze von Anilinen der Formel (VI) mit Diazotierungsmitteln in Gegenwart von Halogenwasserstoffsäuren der Formel

$HW^1$ (VII)

in welcher

$W^1$ für Chlor oder Brom steht,

und in Gegenwart eines Verdünnungsmittels umsetzt und die dabei entstehenden Diazonium-Salze der Formel

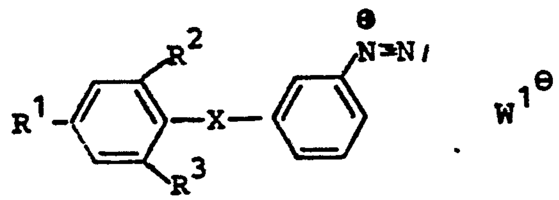

(VIII)

in welcher

$R^1$, $R^2$, $R^3$, X und $W^1$ die oben angegebenen Bedeutungen haben,

mit Verbindungen der Formel

$$CH_2 = \overset{R^{15}}{\overset{|}{C}} - Z^1 \qquad (IX)$$

in welcher

$R^{15}$ und $Z^1$ die oben angegebenen Bedeutungen haben,

in Gegenwart von Halogenwasserstoffsäuren der Formel

$HR^{16}$ (X)

in welcher

$R^{16}$ die oben angegebene Bedeutung hat,

in Gegenwart eines Katalysators und gegebenenfalls in Gegenwart eines Verdünnungsmittels umsetzt,

c) zur Herstellung der Verbindungen der Formel

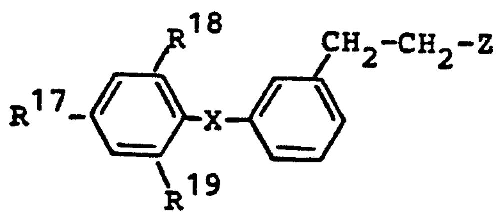

(Ib)

in welcher

X und Z die oben angegebenen Bedeutungen haben,

$R^{17}$ für Halogen oder Trihalogenmethyl steht

und

$R^{18}$ und $R^{19}$ unabhängig voneinander für Wasserstoff, Halogen oder Trihalogenmethyl stehen,

so vorgeht, daß man Verbindungen der Formel

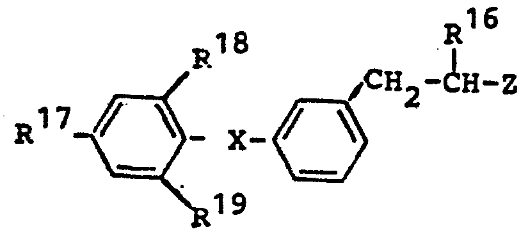

(Ic)

in welcher

$R^{16}$, $R^{17}$, $R^{18}$, $R^{19}$, X und Z die oben angegebenen Bedeutungen haben,

in Gegenwart eines Hydrierungskatalysators mit Wasserstoff gegebenenfalls in Gegenwart eines Verdünnungsmittels umsetzt,

und

d) zur Herstellung von denjenigen substituierten Diphenylethern und Diphenylthioethern der Formel (I),

in denen

$R^4$ für Nitro steht,

so vorgeht, daß man Verbindungen der Formel

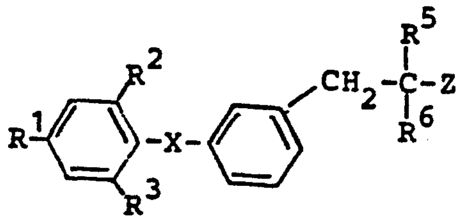

(Id)

in welcher

$R^1$, $R^2$, $R^3$, $R^5$, $R^6$, X und Z die oben angegebenen Bedeutungen haben,

mit einem Nitrierungsmittel gegebenenfalls in Gegenwart eines Katalysators sowie gegebenenfalls in Gegenwart eines Verdünnungsmittels umsetzt.

4. Herbizide Mittel, gekennzeichnet durch einen Gehalt an mindestens einem substituierten Diphenylether oder Diphenylthioether der Formel (I).

5. Verfahren zur Bekämpfung von Unkräutern, dadurch gekennzeichnet, daß man substituierte Diphenylether oder Diphenylthioether der Formel (I) auf die Unkräuter und/oder deren Lebensraum ausbringt.

6. Verwendung von substituierten Diphenylethern und Diphenylthioethern der Formel (I) zur Bekämpfung von Unkräutern.

7. Verfahren zur Herstellung von herbiziden Mitteln, dadurch gekennzeichnet, daß man substituierte Di-

phenylether oder Diphenylthioether der Formel (I) mit Streckmitteln und/oder oberflächenaktiven Stoffen vermischt.

8. Substituierter Diphenylether der Formel

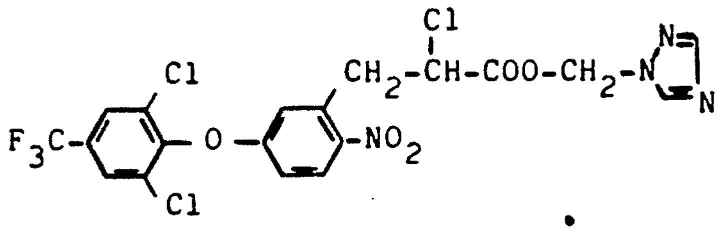

9. Substituierter Diphenylether der Formel

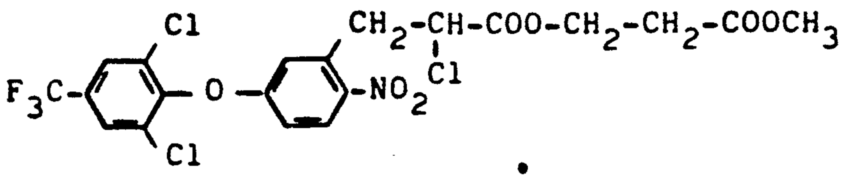

1o. Substituierter Diphenylether der Formel